(19) Europäisches Patentamt
European Patent Office
Office européen des brevets

(11) Numéro de publication: **0 080 934**
**B1**

# (12) FASCICULE DE BREVET EUROPÉEN

(45) Date de publication du fascicule du brevet: **13.01.88**

(21) Numéro de dépôt: **82402137.2**

(22) Date de dépôt: **24.11.82**

(51) Int. Cl.⁴: **C 07 D 311/30**, C 07 D 409/04, A 61 K 31/35, C 07 D 407/04, C 07 D 311/32

ERRATUM

(SEITE, SPALTE, ZEILE)
(PAGE, COLUMN, LINE)
(PAGE, COLONNE, LIGNE)

| DIE TEXTSTELLE :<br>TEXT PUBLISHED :<br>LE PASSAGE SUIVANT : | | | LAUTET BERICHTIGT:<br>SHOULD READ :<br>DEVRAIT ETRE LU : |
|---|---|---|---|
| de benzène. Après élimination de | 7 | 17 | Exemple 9:<br>[oxo-4-phényl-2-4H-[1]-benzopyran -8-yl] acétate d'hydroxy-2-éthyle<br>$C_{19}H_{16}O_5$ (formule 9), PM = 324,32.<br>On place dans un réacteur 4 g d'acide paratoluènesulfonique et 300 ml de benzène. Après élimination de |

Tag der Entscheidung über die Berichtigung / Date of decision on rectification: / Date de décision portant sur modification: 05.10.89

Ausgabe- und Veröffentlichungstag: / Issue and publication date: / Date d'edition et de publication: 29.11.89

Patbl.Nr: / EPB no: / Bull. no: 89/48

(19) Europäisches Patentamt
European Patent Office
Office européen des brevets

(11) Numéro de publication: 0 080 934
B1

# (12) FASCICULE DE BREVET EUROPÉEN

(45) Date de publication du fascicule du brevet: **13.01.88**

(21) Numéro de dépôt: **82402137.2**

(22) Date de dépôt: **24.11.82**

(51) Int. Cl.⁴: **C 07 D 311/30**, C 07 D 409/04, A 61 K 31/35, C 07 D 407/04, C 07 D 311/32

(54) **Acides (oxo-4-4H-(1)-benzopyran-8-yl) alcanoiques, sels et dérivés, préparation et médicaments les contenant.**

(30) Priorité: **25.11.81 FR 8122020**

(43) Date de publication de la demande: **08.06.83 Bulletin 83/23**

(45) Mention de la délivrance du brevet: **13.01.88 Bulletin 88/2**

(84) Etats contractants désignés: **AT BE CH DE FR GB IT LI LU NL SE**

(56) Documents cité:
**FR-A-2 255 894**
**FR-A-2 281 112**
**FR-M-4 092**

**CHEMICAL ABSTRACTS, vol. 78, 1973, pages 460-1, no. 97436t, Columbus Ohio (USA); K. NAKAZAWA et al.: "Synthesis of (+)-fukugetin heptamethyl ether".**

(73) Titulaire: **LIPHA, LYONNAISE INDUSTRIELLE PHARMACEUTIQUE, 34, rue Saint Romain Boîte Postale 8481, F-69359 Lyon Cedex 08 (FR)**

(72) Inventeur: **Briet, Philippe, 216, avenue Félix Faure, F-69003 Lyon (FR)**
Inventeur: **Berthelon, Jean- Jacques, 1, allée des Glycines, F-69150 Decines Charpieu (FR)**
Inventeur: **Collonges, François, Le Grand Champ Chemin de Halage, F-01700 Beynost Miribel (FR)**

(74) Mandataire: **Bouton Neuvy, Liliane, L'Air liquide, Société Anonyme pour L'Etude et L'Exploitation des Procédés Georges Claude 75, Quai d'Orsay, F-75321 Paris Cédex 07 (FR)**

EP 0 080 934 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne des acides [oxo-4-4H-[1]-benzopyran-8-yl] alcanoïques, certaine de leurs sels et dérivés, leurs préparations, des composés intermédiaires nécessaires à leur obtention et des médicaments les contenant.

Ces composés sont représentés par la formule générale

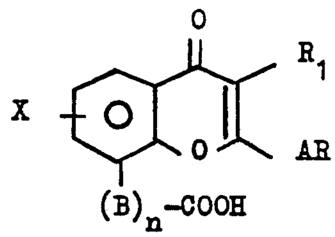

(I)

dans laquelle AR est un radical méthyle, phényle, méthylphenyle, méthoxyphényle, diméthoxyphényle, thényle, furyle, naphtyle, cyclohexyle ou benzyle; B = $CH_2$; $R_1$ est l'hydrogène; X est l'hydrogène, n = 1, ainsi que leurs sels de métaux alcalins, notamment les sels de sodium.

L'invention concerne également les acides représentés par la formule générale I, dans laquelle AR est un radical phényle; $R_1$ est l'hydrogène; X est un radical méthyle; n = 1; B = $CH_2$ ou

$$CH_3-\underset{|}{C}H,$$

ainsi que leurs sels de métaux alcalins, notamment les sels de sodium.

L'objet de l'invention comprend aussi les acides représentés par la formule générale I, dans laquelle AR est un radical phényle; $R_1$ est l'hydrogène; B = $CH_2$; n = 1; X est le radical hydroxy ou méthoxy, ainsi que leurs sels de métaux alcalins.

De plus, l'invention concerne les acides représentés par la formule générale I, dans laquelle AR est un radical phényle; B =

$$CH_3-\underset{|}{C}H,$$

$CH_2$ -CH ou CH = CH; $R_1$ et X sont l'hydrogène; n = 1, ainsi que leurs sels de métaux alcalins.

En outre, l'invention est relative aux acides représentés par la formule générale I, dans laquelle AR est un radical phényle ou hydrogène; n = 1; $R_1$ = un radical phényle; X est l'hydrogène; B = $CH_2$, ainsi que leurs sels de métaux alcalins.

Ces acides peuvent se préparer conformément à l'invention selon l'une ou l'autre des méthodes ci-dessous, qui permettent de les obtenir avec de bons rendements.

Quand dans la formule I des acides: n = 1, et B = $CH_2$, on prépare des acides en hydrolysant un nitrile, de formule:

2

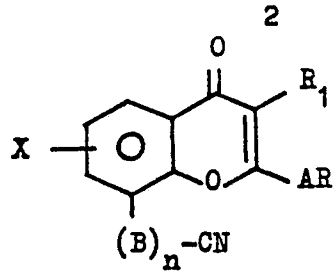

(II)

dans laquelle AR, X et $R_1$ ont les même significations que précédemment, notamment en milieu acide à chaud.

Les nitriles ci-dessus s'obtiennent par traitement du bromométhyl-8 benzopyranone-4 de formule:

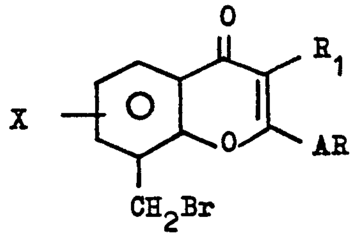

(III)

dans laquelle X, AR et $R_1$ ont les mêmes significations que précédemment, avec un cyanure alcalin.

Quand dans la formule I des acides: n = 1 et B = CH = CH on peut aussi mettre en réaction un bromométhyl-8 benzopyranone-4 de formule (III) avec l'hexaméthylènetétramine, puis procéder à la condensation de l'aldéhyde obtenu. Quand dans la formule I des acides: n =1 et B = CH = CH, on réalise la dite condensation avec l'anhydride acétique en présence d'acétate de sodium. Les [oxo-4-phényl-2 et 3-4H-[1]-benzopyran-8-yl] carboxaldéhyde sont des produits intermédiaires nouveaux. Quand AR = H et $R_1$ = phényle on procède à la condensation de l'aldéhyde obtenu avec le diéthylaminométhylènediphosphonate de tétraéthyle en présence d'hydrure de sodium. Par hydrolyse on obtient l'acide correspondant. L'[oxo-4 phényl-3-4H-[1] -benzopyran 8-yl] carboxaldéhyde constitue un produit intermédiaire.

Dans le cas où n = 1 et B est le radical alkyle ramifié

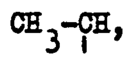

l'introduction de la ramification est réalisée par transformation de l'acide en ester puis en malonate qui est alcoylé puis hydrolysé en acide correspondant. Quand n = 1 et B est $CH_2$ - $CH_2$, on fait réagir dans un premier temps une bromométhyl-8 benzopyranone-4 de formule III avec le malonate de diéthyle en présence d'un agent basique comme l'hydrure de sodium, un alcoolate de sodium ou le sodium dans un solvant approprié. Le malonate substitué obtenu est ensuite hydrolysé en acide correspondant.

Les nitriles représentés par la formule II dans laquelle AR est un radical méthyle, phényle, méthylphényle, méthoxyphényle, diméthoxyphényle, benzyle, cyclohexyle, thényle, furyle, naphtyle, $R_1$ et X sont l'hydrogène et les nitriles représentés par la même formule II que ci-dessus dans laquelle AR est le radical phényle, $R_1$ est l'hydrogène, X un radical méthyl ou méthoxy et aussi les nitriles représentés par la même formule II dans laquelle AR est le radical phényle, $R_1$ le radical phényle et X l'hydrogène sont des composés intermédiaires nouveaux utilisables dans la préparation des acides de l'invention.

Les malonates représentés par les formules

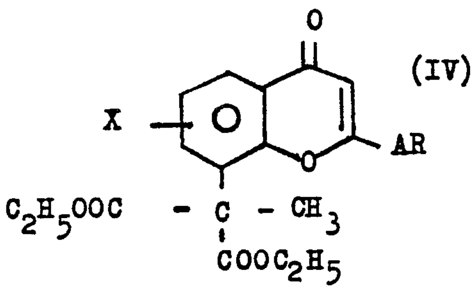

(IV) et

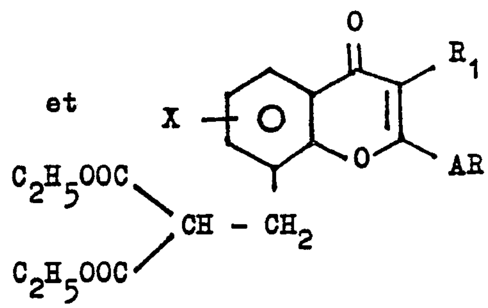

(V)

dans lesquelles pour la formule (IV) X désigne le radical méthyle, $R_1$ est l'hydrogène et AR le radical phényle, et pour la formule (V), X désigne l'hydrogène, $R_1$ l'hydrogène et AR le radical phényle, sont des composés intermédiaires nouveaux dans la préparation des composés de l'invention.

Les sels de métaux alcalins des acides selon l'invention, notamment les sels de sodium s'obtiennent par neutralisation des acides précédents.

Les dérivés des acides sous forme d'esters et aminoesters de formule (VI)

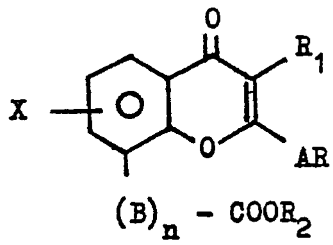

(VI)

dans laquelle AR est un radical phényle, $R_1$ = hydrogène, X est l'hydrogène, n = 1, B = $CH_2$, $R_2$ est un radical méthyle, éthyle, hydroxyéthyle, diéthylaminoéthyle, diméthylaminopropyle ou morpholinoéthyle; ainsi que les esters et aminoesters représentés par la formule VI dans laquelle AR est un radical thényle, $R_1$ est l'hydrogène, n = 1, B = $CH_2$, $R_2$ est un radical méthyle ou diéthylaminoéthyle; et les esters et aminoesters de formule VI dans laquelle AR est un radical phényle, $R_1$ est l'hydrogène, n = 1, B = $CH_2$, X est le radical méthyle et $R_2$ un radical méthyle ou diéthylaminoéthyle; l'aminoester de formule VI dans laquelle AR est un radical phényle, $R_1$ et X sont l'hydrogène, B = CH = CH, n = 1 et $R_2$ est un radical diéthylaminoéthyle ou morpholinoéthyle; l'aminoester de formule VI dans laquelle AR est un radical phényle, $R_1$ = hydrogène, X un radical méthoxy, B = $CH_2$, n=1 et $R_2$ est le radical diéthylaminoéthyle; les aminoesters de formule VI dans laquelle AR est un radical méthylphényle ou méthoxyphényle, $R_1$ et X sont l'hydrogène, B = $CH_2$, n = 1 et $R_2$ est le radical diéthylaminoéthyle, et l'aminoester de formule VI dans laquelle AR est un radical phényle, $R_1$ est l'hydrogène, B =

$$CH_3-CH\ ;$$

n = 1; X est l'hydrogène et $R_2$ est le radical diéthylaminoéthyle sont des composés nouveaux et font à ce titre partie de l'invention.

Les esters se préparent par estérification des acides précédents, et les aminoesters sont obtenus par condensation des dits acides précédents avec un halo, de préférence chloroalkylaminodialkyle correspondant ou la chloroéthylmorpholine.

Le N-[N',N'-diéthylamino-2 éthyl] [oxo-4-phényl-2-4H-[1]-benzopyran-8-yl] acétamide obtenu par condensation de l'[oxo-4-phényl-2-4H-[1]-benzopyran-8-yl] acétate d'éthyle avec la diéthylamino-2 éthylamine, constitue un dérivé faisant partie de l'invention.

Les sels des esters, aminoesters et amides avec des acides minéraux ou organiques acceptables en thérapeutique humaine sont d'un accès facile et d'une utilisation aisée.

Les acides et leurs dérivés selon l'invention et plus particulièrement le produit de l'exemple 3 ont montré d'intéressantes propriétés pharmacologiques dans le domaine anti-tumoral. Les tests pharmacologiques ont été effectués sur plusieurs types de tumeurs. A titre d'exemple sur;;

<u>La leucémie lymphocytique P 388</u>: le test est effectué sur des souris de souche CFD1. On implante intrapéritonalement 0,1 ml de fluide ascitique contenant $10^6$ cellules, au jour zéro. Les animaux sont traités en i.p. du jour 1 au jour 9, avec une injection par jour et sont pesés au jour 1 et au jour 5 (jour de toxicité). La valeur de contrôle (médiane de survie) acceptée est de 9-14 jours. On utilise 6 souris par test (un test = une dose). Une différence de poids de plus de 4g au jour 5 entre les souris tests et les souris contrôles est une indication de toxicité. On détermine le rapport

$\frac{T}{C} = \frac{\text{médiane de survie des souris tests}}{\text{médiane de survie des souris contrôles}}$ Un T/C < 85 % indique une toxicité.

Un T/C situé entre 85 et 120 % indique une non-activité.

Un T/C > 120 % indique une activité.

Dans ce test le produit de l'exemple 3, administré à 200 mg/ kg a donné un $\frac{T}{C}$=195. Dans les mêmes conditions le 5-Fluorouracile, agent anticancéreux de la classe des antimétabolites (antipyrimidine) a donné un $\frac{T}{C}$=185.

<u>Le carcinome 38 du colon</u>: Ce test est effectué sur des souris de souche B6C3F1 ou BDF1. On implante en sous cutané un fragment de 70 mg de tumeur au jour zéro. Les animaux sont traités en i.p. du jour 2 au jour 9 (2 injections). La valeur de contrôle acceptée est le poids de tumeur moyen situé entre 400 et 2000 mg. On utilise 10 souris par test. On détermine le rapport:

$\frac{T}{C} = \frac{\text{poids de tumeur moyen du test}}{\text{poids de tumeur moyen du contrôle}}$

Une activité est indiquée par un $\frac{T}{C}$ inférieur à 42.

A titre d'exemple, le produit de l'exemple 3 a donné dans les trois essais les résultats suivants: Doses administrées $\frac{T}{C}$ 200 mg/kg: 10; 200 mg/kg: 0 ; 50 mg/kg: 38.

Le médicament contenant comme principe actif un composé de l'invention, associé à un véhicule ou un excipient pharmaceutique acceptable est présenté sous une forme adaptée en vue d'une administration orale, parentérale ou intra-veineuse.

Les formes posologiques sont unitaires telles que des dragées, comprimés, capsules, gélules, ampoules ou flacons; Ces formes posologiques renferment entre 50 et 1000 mg de substance active.

A titre d'exemple on trouvera les compositions suivantes:

Comprimé enrobé: Principe actif: 100 mg. Excipients: stéarate de magnésium, lactose, talc, amidon, acide alginique, hydroxypropylcellulose.

Flacon: Principe actif: 1000 mg sous forme lyophilisée reprise par 20 ml d'eau pour préparation injectable.

Il est donné ci-après des exemples qui illustrent l'invention à titre non limitatif.

**Exemple 1:**

[/oxo-4-phényl-2-4H [1] -benzopyran-8-yl] acétonitrile $C_{17}H_{11}NO_2$ (formule 1), PM = 261,26.

On porte une solution de 31 g (0,476 mole) de cyanure de potassium dans 150 ml d'eau à 60-70°C. On ajoute ensuite par fraction une suspension de 75 g (0,238 mole) de bromométhyl-8-phényl-2-4H-[1]-benzopyran-4-one dans 965 ml d'éthanol bouillant. On porte ensuite 3 heures à reflux. On filtre à chaud et le filtrat est placé au réfrigérateur 48 heures; Le précipité formé est essoré, séché. Poids obtenu: 42 g (Rdt 67,5 %), IR:ν c = 0 : 1640 $cm^{-1}$. RMN (DMSO)δ par rapport au T.M.S. 2H à 4,45 (singulat), 1 H à 7,05 (singulet), 8 H de 7,15 à 8,6 (massif).

**Exemple 2:**

Acide [oxo-4-phényl-2-4H-[1] -benzopyran-8-yl]-acéticue $C_{17}H_{12}O_4$ (formule 2) PM = 280,27

On place 42 g (0,16 mole) d'oxo-4-phényl-2-4H-[1]-benzopyran-8-yl] acétonitrile dans un mélange formé de 117 ml d'acide acétique et 117 ml d'eau. On ajoute ensuite lentement 117 ml d'acide sulfurique concentré et on porte à reflux 3 heures 30. On refroidit, on verse dans 1,5 litre d'eau glacée. Le précipité formé est essoré, repris par une solution de bicarbonate de sodium à 5 % à 50-60°C. On filtre le léger insoluble et on acidifie avec de l'acide chlorhydrique concentré. Le précipité beige est essoré, lavé à l'eau, séché et recristallisé dans l'alcool. Poids obtenu: 39,7 g (Rdt 88,6 %), $PF_G$ : 234°C, IR : ν = O (pyrone) : 1640 $cm^{-1}$; ν = O (acide) : 1720 $cm^{-1}$, RMN (DMSO) δ en ppm par rapport au T.M.S. 2H à 4 (singulet), 1 H à 7 (singulet) 8 H de 7,3 à 8,3 (massif), 1 H à 12,6 (échangeable avec $D_2O$).

Analyse pondérale:

| | C % | H % | O % |
|---|---|---|---|
| Calculée | 72,84 | 4,32 | 22,84 |
| Trouvée | 72,73 | 4,36 | |

**Exemple 3:**

[oxo-4-phényl-2-4H-[1]-benzopyran-8-yl]-acétate de diéthylamino-2- éthyle $C_{23}H_{25}NO_4$ (formule 3) PM = 379,39.

On solubilise à chaud 21 g (0,075 mole) d'acide [oxo-4-phényl-2-4H-[1]-benzopyran-8-yl-]-acétique dans 1,2 litre d'éthanol. On arrête le chauffage et on ajoute une solution de 4,2 g (0,075 mole) de potasse dans 75 $cm^3$ d'éthanol. On agite trente minutes à température ambiante, on évapore l'alcool, on sèche le résidu solide azéotropiquement avec du benzène, on ajoute ensuite 370 ml d'acétone, puis une solution de 11 g (0,0813 mole) diéthylamino-2-chloro-1-éthane dans 37 ml d'acétone. On porte 4 heures au reflux. On filtre l'insoluble à chaud et on évapore sous vide. On obtient une huile qui cristallise à température ambiante. IR: ν = O (ester) : 1730 $cm^{-1}$ ν = O (pyrone): 1660 $cm^{-1}$.

Chlorhydrate: $C_{23}H_{26}ClNO_4$, PM = 415,89, PFG: 186°C (isopropanol) IR : ν c = O (ester) : 1740 $cm^{-1}$, ν c = O (pyrone) : 1660 $cm^{-1}$, ν NH⊕: bande de 2400 à 2700 $cm^{-1}$, RMN ($CDl_3$) δ en ppm par rapport au T.M.S. 6 H à 1,2 (triplet) 6 H de 2,8 à 3,4 (massif), 2 H à 4,15 (singulet), 2 H à 4,6 (triplet), 1 H à 6,8 (singulet), 8 H de 7,2 à 8,3 (massif), 1 H à 12,6 (échangeable avec $D_2O$).

Analyse pondérale:

| | C % | H % | Cl % | N % | O % |
|---|---|---|---|---|---|
| Calculée: | 66,42 | 6,30 | 8,52 | 3,36 | 15,40 |
| Trouvée: | 66,42 | 6,42 | 8,60 | 3,40 | |

**Exemple 4**:

[oxo-4-phényl-2-4H-[1]-benzopyran-8yl] acétate de (morpholinyl-4)-2- éthyle $C_{23}H_{23}NO_5$ (formule 4), PM = 393,38.

Préparé selon le procédé de l'exemple 3 à partir de 17,5 g (0,0625 mole) d'acide [oxo-4-phényl-2-4H-[1] -benzopyran-8-yl] acétique, 3,9 g (0,0625 mole) de potasse et 11,3 g (0,0755 mole) de (chloro-2-éthyl) -4-morpholine. Après isolement et traitement par HCl gazeux et recristallisation dans le méthanol on obtient un solide blanc.

Chlorydrate: $C_{23}H_{24}ClNO_3$, PM = 429,88

Poids obtenu: 10,1 g (Rdt: 37,3 %), $PF_G$ = 193-194° C.

IR:ν c = O (ester) : 1740 $cm^{-1}$, ν c = O (pyrone): 1640 $cm^{-1}$, ν NH ⊕: bande de 2300 à 2600 $cm^{-1}$;

RMN (DMSO) δ en ppm par rapport au TMS.

6 H de 2,8 à 3,5 (massif), 4 H de 3,7 à 4,1 (massif), 2 H à 4,2 (singulet), 2 H à 4,55 (triplet), 1 H à 6,8 (singulet), 8 H de 7,2 à 8,3 (multiplet), 1 H à 12,5 (échangeable avec $D_2O$).

**Exemple 5**:

[oxo-4-phényl-2-4H-[1]-benzopyran-8-yl-] acétate de diméthylamino-3 propyle $C_{2223}NO_4$ (formule 5), PM = 365,38.

Préparé selon le procédé de l'exemple 3 à partir de 12,3 g (0,044 mole) d'acide [oxo-4-phényl-2-4H-[1]-benzopyran-8-yl] acétique, 2,74 g (0,044 mole) de potasse et 6,45 g (0,053 mole) de diméthylamino-3-chloro-1-propane. Après traitement on obtient un solide blanc. Poids obtenu: 10,5 g (Rdt: 65,31 %), $PF_G$: 112° C (diisopropyléther). IR: ν c = O (ester): 1735 $cm^{-1}$, ν c = O (pyrone) : 1645 $cm^{-1}$

RMB ($CDCl_3$) ⊕ en ppm par rapport au TMS.

10 H de 1,6 à 3,2 (multiplet), 4 H de 3,9 à 4,2 (multiplet) 1 H à 6,8 (singulet), 8 H de 7,2 à 8,3 (multiplet).

Chlorhydrate: $C_{22}H_{24}ClNO_4$, PM = 401,88, $PF_G$: 162-164° C (acétone).

Analyse pondérale:

| | C % | H % | Cl % | N % | O % |
|---|---|---|---|---|---|
| Calculée: | 65,75 | 6,02 | 8,82 | 3,49 | 15,92 |
| Trouvée: | 65,38 | 6,17 | 8,71 | 3,47 | |

**Exemple 6**:

[oxo-4-phényl-2-4H-[1]-benzopyran-8-yl-] acétate d'éthyle $C_{19}H_{16}O_4$ (formule 6) PM = 308.

On porte à reflux pendant 7 heures 5,1 g (0,0175 mole) d'acide [oxo-4-phényl-2-4H-[1]-benzopyran-8yl] acétique dans 75 ml d'éthanol absolu en présence de 10 ml d'acide sulfurique concentré. Le milieu est ensuite placé au réfrigérateur, le précipité formé est essoré, lavé avec une solution de bicarbonate et à l'eau. On le recristallise dans l'éthanol. Poids obtenu: 4 g (Rdt: 74 %), $PF_K$ : 140° C.

IR: ν c = O (ester) : 1740 $cm^{-1}$, ν c = O (pyrone): 1645 $cm^{-1}$

**Exemple 7**:

[oxo-4-phényl-2-4H-[1]-benzopyran-8-yl-] acétate de méthyle $C_{18}H_{14}O_4$ (formule 7), PM = 294.

Préparé selon l'exemple 6 à partir de 18 g (0,064 mole) d'acide [oxo-4-phényl-2-4H-[1]-benzopyran-8-yl] acétique, 260 ml de méthanol anhydre et 13 ml d'acide sulfurique concentré. Poids obtenu : 18,1 g (Rdt: 96 %), $PF_K$: 168 - 169° C (méthanol)

IR : ν c = O (ester) : 1735 $cm^{-1}$, ν c = O (lactone): 1640 $cm^{-1}$

RMN ($CDCl_3$) δ en ppm par rapport au TMS.

3 H à 3,8 (singulet), 2 H à 4,1 (singulet) 1 H à 6,9 (singulet)

8 H de 7,4 à 8,55 (multiplet).

**Exemple 8**:

sel de sodium de l'acide [oxo-4-phényl-2-4H-[1]-benzopyran-8-yl] acé- tique $C_{17}H_{11}O_4Na$, (formule 8), PM = 302,24.

On prépare une solution de 2,1 g (0,025 mole) de bicarbonate de sodium dans 400 ml d'eau. On porte à 50°C et on ajoute 7 g (0,025 mole) d'acide [oxo-4-phényl-2-4H-[1]-benzopyran-8-yl] acétique. Après solubilisation complète on laisse revenir à 30°C et on verse dans 2 litres d'acétone. Un précipité blanc se forme, on l'essore, le rince à l'acétone et le sèche. Poids obtenu 6,2 g (Rdt: 79,68 %), PFG = 302-304°C (diméthylformamide). IR: ν c = O (pyrone): 1640 $cm^{-1}$.

Analyse pondérale Hémihydrate PM = 311,24

| | C % | H % | O % | Na % |
|---|---|---|---|---|
| Calculée: | 65,54 | 3,85 | 23,13 | 7,38 |
| Trouvée: | 65,60 | 3,88 | | |

de benzène. Après élimination de l'eau, on ajoute 28 g (0,1 mole) d'acide [oxo-4-phényl-2-4H-[1]-benzopyran-8-yl] acétique et 300 ml d'éthylène glycol. On porte 4 heures à reflux avec élimination azéotropique de l'eau. La solution orange obtenue est concentrée sous vide et le résidu est versé sur 1,5 litre d'eau glacée. Le précipité beige formé est essoré, lavé à l'eau et recristallisé dans 300 ml d'éthanol. Poids obtenu: 23,5 g (Rdt: 72,5 %), $PF_G$ = 158-159°C. IR : ν c = O (pyrone): 1640 $cm^{-1}$, ν c = O (ester) : 1725 $cm^{-1}$, ν OH : 3400 $cm^{-1}$; RMN ($CDCl_3$) en ppm par rapport au TMS.

3 H de 3,45 à 3,9 : (multiplet, 1H échangeable avec $D_2O$), 4 H de 3,9 à 4,4 : (multiplet), 1 H à 6,8 : (singulet), 8 H de 7,3 à 8,3 : (multiplet).

Analyse pondérale:

| | C % | H % |
|---|---|---|
| Calculée: | 70,36 | 4,97 |
| Trouvée: | 70,26 | 4,74 |

**Exemple 10**:

N - [N', N'-diéthylamino-2-éthyl]-[oxo-4-phényl-2-4H-[1]-benzopyran-8- yl] acétamide $C_{23}H_{26}N_2O_3$ (formule 10), PM = 378,43.

On porte 5 heures à 135-140°C un mélange de 14,8 g (0,048 mole) d'[oxo -4-phényl-2-4H-[1]-benzopyran-8-yl] acétate d'éthyle et 6,2 g (0,053 mole) de diéthylamino-2 éthylamine. On évapore ensuite sous vide et le résidu est repris par l'hexane à chaud puis recristallisé dans l'acétate d'éthyle. On obtient ainsi: 7,5 grammes (Rdt: 41,28 %) IR : ν c = O (pyrone et amide): 1640-60 $cm^{-1}$, ν NH : 3300 $cm^{-1}$

Chlorhydrate $C_{23}H_{27}ClN_2O_3$, PM = 414,93, $PF_G$ = 216-218°C (éthanol).

Analyse pondérale

| | C % | H % | N % |
|---|---|---|---|
| Calculée: | 66,57 | 6,56 | 6,75 |
| Trouvée: | 66,44 | 6,66 | 6,76 |

**Exemple 11**:

[oxo-4- (thényl-2)-2-4H-[1]benzopyran-8yl] acétonitrile $C_{15}H_9NO_2S$, (formule 11), PM = 267.

Préparé selon l'exemple 1 à partir de 69,5 g (0,215 mole) de bromométhyl-8- (thényl-2)-2-4H-[1]-benzopyran-4-one et 28,4 g (0,42 mole) de cyanure de potassium. Poids obtenu : 21,7 g (Rdt: 40 %) $PF_K$ : 184°C (Ethanol). IR : ν c = O (pyrone) : 1645 $cm^{-1}$, ν c ≡ N : 2150 $cm^{-1}$ RMN (DMSO) δ en ppm par rapport au TMS, 2 H à 4,55 (singulet), 1 H à 7 (singulet), 6 H de 7,3 à 8,3 (multiplet).

**Exemple 12**:

Acide [oxo-4- (thényl-2)-2-4H-[1]-benzopyran-8-yl] acétique $C_{15}H_{10}O_4S$ (formule 12), PM = 286,31.

Préparé selon l'exemple 2 à partir de 21,7 g (0,081 mole) d'[oxo-4-(thényl-2)-2-4H-[1]-benzopyran-8-yl] acétonitrile. On obtient après traitement au bicarbonate un produit que l'on recristallise dans le dioxanne. Poids obtenu : 13,9 g (Rdt 60 %), $PF_G$ = 247-255°C. IR : ν c = O (pyrone) : 1630 $cm^{-1}$, ν c = O (acide) : 1710 $cm^{-1}$, ν OH: 2400 - 2800 $cm^{-1}$, RMN (DMSO) δ en ppm par rapport au TMS, 2 H à 4 (singulet), 1 H à 7 (singulet), 6 H de 7,3 à 8,2 (multiplet), 1 H à 12 (massif échangeable avec $D_2O$).

**Exemple 13**:

[oxo-4-(thényl-2)-2-4H-[1]-benzopyran-8-yl] acétate de méthyle. $C_{16}H_{12}O_4S$, (formule 13), PM = 300,32.

Préparé selon l'exemple 7 à partir de 10,2 g (0,035 mole) d'acide [oxo-4-(thényl-2)-2-4H-[1]-benzopyran-8-yl] acétique. Poids obtenu : 7,7 g (Rdt: 68,5 %), $PF_G$ = 168-170°C (méthanol), IR : ν c = O (Pyrone) : 1645 $cm^{-1}$, ν c = O (ester): 1720 $cm^{-1}$.

Analyse pondérale:

| | C % | H % | S % |
|---|---|---|---|
| Calculée: | 63,98 | 4,03 | 10,68 |
| Trouvée: | 64,07 | 4,02 | 10,70 |

**Exemple 14**:

[Oxo-4-(thényl-2)-4H-[1]-benzopyran-8yl] acétate de ('-N diéthylamino) -2-éthyle. $C_{21}H_{23}NO_4S$ (formule 14). PM = 385,45.

Préparé selon l'exemple 3 à partir de 9,6 g (0,036 mole) d'acide [Oxo-4-(thényl-2)-2-4H-[1]-benzopyran-8-yl] acétique, 2,2 g (0,033 mole) de potasse et 5 g (0,033 mole) de chloro-2-N,N-diéthylethylamine. Après traitement on obtient une huile. Poids obtenu : 12 g (Rdt: 88 %).

Chlorhydrate $C_{21}H_{24}ClNO_4S$. PM = 421,92. $PF_G$ = 210-213°C (éthanol)

IR : ν NH ⊕ = 2800 - 2400 $cm^{-1}$, ν c = O (ester) = 1760 $cm^{-1}$, ν c = O (pyrone) = 1640 $cm^{-1}$.

RMN ($CDCl_3$) δ en ppm par rapport au TMS, 6 H à 1,15 (triplet, J = 7 Hz), 6 H de 2,7 à 3,5 (multiplet), 2 H à 4,1 (singulet), 2 H à 4,45 (triplet, J = 6 Hz), 1 H à 6,9 (singulet), 6 H de 7,1 à 8 (multiplet), 1 H à 10,6 (échangeable avec $D_2O$),

Analyse

| | C % | H % | Cl % | N % | O % | S % |
|---|---|---|---|---|---|---|
| Calculée: | 59,80 | 5,73 | 8,40 | 3,31 | 15,16 | 7,60 |
| Trouvée: | 59,85 | 5,79 | | 3,35 | | 7,67 |

**Exemple 15**:

[méthyl-6-oxo-4-phényl-2-4H-[1]-benzopyran-8-yl] acétonitrile $C_{18}H_{13}NO_2$ (formule 15), PM = 275.

Préparé selon l'exemple 1 à partir de 16,4 g (0,05 mole) de bromométhyl-8-méthyl-6-4H[1]-benzopyran-4-one et 6,5 g (0,1 mole) de cyanure de potassium. Poids obtenu : 11 g (Rdt: 80 %), $PF_K$ : 255°C. IR : ν c = O (pyrone) : 1630 $cm^{-1}$, ν CN : 2250 $cm^{-1}$.

**Exemple 16**:

Acide [méthyl-6-oxo-4-phényl-2-4H-[1]-benzopyran-8-yl] acétiqgue $C_{18}H_{14}O_4$, (formule 16), PM = 294.

Préparé selon l'exemple 2 à partir de 11 g (0,04 mole) de [méthyl-6-oxo-4-phényl-2-4H-[1]-benzopyran-8-yl] acétonitrile. Poids obtenu 6,2 g (Rdt 53 %), $PF_K$ : 238-239°C (éthanol). IR: ν c = O (pyrone) 1640 $cm^{-1}$, ν c = O (acide) 1710 $cm^{-1}$. RMN (DMSO) δ en ppm par rapport au TMS : 3 H à 2,4 (singulet), 1 H à 3,8 (massif échangeable avec $D_2O$) 2 H à 4 (singulet), 1 H à 7 (singulet), 7 H de 7,4 à 8,3 (multiplet).

**Exemple 17**:

[méthyl-6-oxo-4-phényl-2-4H-[1]-benzopyran-8-yl] acétate de méthyle $C_{19}H_{16}O_4$, (formule 17), PM = 308.

Préparé selon l'exemple 7 à partir da 84,7 g (0,288 mole) d'acide [méthyl-6-oxo-4-phényl-2-4H-[1]-benzopyran-8-yl] acétique. Poids obtenu : 75 g (Rdt: 84,55 %), $PF_K$ : 228-230°C. IR : ν c = O (ester) 1740 $cm^{-1}$, ν c = O (pyrone) : 1638 $cm^{-1}$.

**Exemple 18**:

(Méthyl-6-oxo-4-phényl-2-4H-[1]-benzopyran-8-yl) acétate de )N, N- diéthylamino)-2-éthyle. $C_{24}H_{27}NO_4$ (formule 18), P.M. = 393,49.

Préparé selon l'lexemple 3 à partir de 8,5 g (0,029 mole) d'acide (méthyl-6-oxo-4-phényl-2-4H-[1]-benzopyran-8-yl) acétique et de 4,73 g (0,035 mole) da chloro-2-N,N-diéthyléthylamine en remplaçant l'acétone par le DMF et en chauffant 2 h30 à 80°. Après évaporation du DMF, on reprend le résidu au chloroforme, lave à la soude N, puis à l'eau, sèche, évapore sous vide et recristallise dans un mélange hexane-toluène. Poids obtenu : 8,8 g (Rdt : 77 %), $PF_K$ = 120°C, IR : ν c = O (pyrone) = 1655 $cm^{-1}$; ν c = O (ester) = 1750 $cm^{-1}$, RMN ($CDCl_3$) 6 H à 0,92 (triplet, J = 7 Hz), 9 H de 2,3 à 2,9 (multiplet), 2 H à 3,97 (singulet), 2 H à 4,2 (triplet, J = 6 Hz), 1 H à 6,8 (singulet), 7 H de 7,2 à 8,1 (multiplet).

Chlorhydrate $C_{24}H_{28}ClNO_4$. PM = 429,95, $PF_G$ = 188-190° (éthanoléther).

Analyse pondérale

| | C % | H % | Cl % | N % | O % |
|---|---|---|---|---|---|
| Calculée: | 64,07 | 6,56 | 8,25 | 3,26 | 14,89 |
| Trouvée: | 64,34 | 6,35 | 8,27 | 3,46 | |

**Exemple 19**:

[oxo-4-phényl-2-4H-[1]-benzopyran-8-yl]-2, méthyl-2-malonate de dié- thyle, $C_{23}H_{22}O_6$ (formule 19), PM = 394.

Dans un réacteur on place 200 ml de carbonate d'éthyle et on ajoute 3,26 g (0,068 mole) d'hydrure de sodium à 50 %. On porte le milieu à 60°C et on additionne 19 g (0,065 mole) d'[oxo-4-phényl-2-4H-[1]-benzopyran-8-yl] acétate de méthyle. Puis on porte progressivement au reflux et au bout d'une heure un précipité jaune se forme. On poursuit le reflux 2 heures puis on refroidit à 20°C et on additionne à cette température une solution de 23 g d'iodure de méthyle dans 65 ml de diméthyl-formamide. On laisse sous agitation une nuit, on essore le précipité et le filtrat est évaporé sous vide, repris par l'eau et extrait au benzène. Après séchage et évaporation du solvant, l'huile obtenue est recristallisée dans le diisopropyléther. Poids obtenu : 19,3 g (Rdt 75 %) $PF_K$ : 130°C. RMN ($CCl_4$) δ en ppm par rapport au TMS, 6 H à 1,2 (triplet), 3 H à 2 (singulet), 4 H à 4,15 (quartet), 1 H à 6,85 (singulet), 8 H de 7,3 à 8,4 (multiplet).

**Exemple 20**:

Acide [oxo-4-phényl-2-4H-[1]-benzopyran-8-yl]-2 propionique $C_{18}H_{14}O_4$ (formule 20) PM = 294.

On porte à reflux 7 heures 19 g (0,048 mole) d'[oxo-4-phényl-2-4H-[1]-benzopyran-8-yl]-2, méthyl-2, malonate de diéthyle, 100 ml d'acide acétique et 50 ml d'acide chlorhydrique concentré. On refroidit à température ambiante et on ajoute sous agitation 50 ml d'eau, on filtre le précipité formé, on le lave à l'eau et on le reprend par 500 ml d'une solution à 5 % de bicarbonate de soude, on filtre l'insoluble et on acidifie avec HCl concentré. Le précipité blanc est essoré et recristallisé dans l'acétate d'éthyle. Poids obtenu : 4,1 g (28,9 %). $PF_G$ 216-218°C. IR : ν c = O (pyrone) : 1640 $cm^{-1}$, ν c = O (acide) : 1740 $cm^{-1}$. RMN (DMSO) δ en ppm par rapport au TMS 3 H à 1,65 (doublet), 1 H à 3,8 (échangeable avec $D_2O$), 1 H à 4,4 (quartet), 1 H à 7,1 (singulet), 8 H de 7,4 à 8,4 (multiplet).

Analyse pondérale:

| | C % | H % |
|---|---|---|
| Calculée: | 73,46 | 4,79 |
| Trouvée: | 73,47 | 4,70 |

393,46.

Préparé selon l'exemple 3 à partir de 3,6 g (0,012 mole) d'acide [oxo-4-phényl-2-4H-[1]-benzopyran-8-yl]-2-propionique, 0,8 g (0,012 mole) de potasse et 1,82 g (0,012 mole) de chloro-2-N,N-diéthyléthylamine. On obtient une huile.

Chlorhydrate $C_{24}H_{28}ClNO_4$, PM = 429,91, $PF_G$ = 261-3°C (éthanol) IR : ν NH⊕ = 2400 - 2800 $cm^{-1}$, ν c = O (ester) = 1745 $cm^{-1}$, ν c = O (pyrone) = 1660 $cm^{-1}$. RMN ($CDCl_3$) en ppm par rapport au TMS, 6 H à 1,2 (triplet J = 7 Hz), 3 H à 1,8 (doublet J = 8 Hz), 6 H de 2,4 à 3,3 (multiplet), 3 H de 3,4 à 3,8 (multiplet), 1 H à 6,9 (singulet), 8 H de 7,2 à 8,4 (multiplet), 1 H à 12,7 (échangeable avec $D_2O$).

Analyse

| | C % | H % | Cl % | N % | O % |
|---|---|---|---|---|---|
| Calculée: | 67,04 | 6,56 | 8,25 | 3,26 | 14,89 |
| Trouvée: | 66,88 | 6,52 | | 3,20 | |

**Exemple 22**:

[méthyl-6-oxo-4-phényl-2-4H-[1]-benzopyran-8-yl]-2-méthyl-2, malonate de diéthyle $C_{24}H_{24}O_6$ (formule 22) PM = 408,45.

Préparé selon l'exemple 19 à partir de 31,3 g (0,1 mole) de [méthyl-6-oxo-4-phényl-2-4H-[1]-benzopyran-8-yl] acétate de méthyle, 325 ml de carbonate d'éthyle, 5,39 g (0,11 mole) d'hydrure de sodium à 50 %, et 37,2 g d'iodure de méthyle. Poids obtenu : 32,3 g (Rdt : 77,8 %), $PF_K$ 147-148°C (diisopropyléther). RMH ($CDCl_3$) δ en ppm par rapport au TMS, 6 H à 1,2 (triplet, 3 H à 2,05 (singulet), 3 H à 2,5 (singulet, 4 H à 4,2 (quartet), 1 H à 6,8 (singulet), 7 H de 7,2 à 8 (multiplet).

**Exemple 23**:

Acide [méthyl-6-oxo-4-phényl-2-4H-[1]-benzopyran-8-yl]-2, propionique $C_{19}H_{16}O_4$ (formule 23), PM = 308,32.

Préparé selon l'exemple 20 à partir de 32 g (0,078 mole) de [méthyl-6-oxo-4-phényl-2-4H-[1]-benzopyran-8-yl]-2 méthyl-2-malonate de diéthyle. Poids obtenu : 8,5 g (Rdt : 35 %). $PF_G$ = 232-234°C (éthanol-eau). RMN (DMSO) δ en ppm par rapport au TMS, 3 H à 1,6 (doublet), 3 H à 2,4 (singulet), 1 H à 4,3 (quartet), 1 H à 4,6 (massif étalé échangeable avec $D_2O$), 1 H à 7 (singulet), 7 H de 7,4 à 8,3 (multiplet).

Analyse pondérale:

| | C % | H % |
|---|---|---|
| Calculée: | 74,01 | 5,23 |
| Trouvée: | 74,04 | 5,21 |

**Exemple 24**:

[oxo-4-phényl-2-4H-[1]-benzopyran-8-yl] carboxaldéhyde $C_{16}H_{10}O_3$ (formule 24), PM = 250,26.

Méthode A

On place dans un réacteur 37,2 g (0,118 mole) de bromométhyl-8-phényl -2-4H-[1]-benzopyran-4-one, 27,6 g (0,197 mole) d'hexaméthylène tétramine et 400 ml de chloroforme. On porte le milieu 2 h à reflux, le chloroforme est évaporé sous vide et 515 ml d'acide acétique sont ajoutés. L'ensemble est porté 2 h à reflux, puis après filtration et addition de 67,5 ml d'acide chlorhydrique concentré, le reflux est poursuivi pendant 30 minutes. On laisse au repos une nuit. Après évaporation sous vide, le résidu est recristallisé dans le mélange eau-acide acétique. Poids obtenu : 11,6 g (Rdt : 39,3 %), $PF_K$ : 190°C. IR : ν c = O (pyrone) : 1650 $cm^{-1}$, ν c = O (aldéhyde): 1710 $cm^{-1}$. RMN (DMSO) δ en ppm par rapport au TMS, 1 H à 7,2 (singulet), 8 H de 7,5 à 8,5 (multiplet), 1 H à 10,7 (singulet).

Méthode B

Dans un réacteur on place 10,85 g (0,043 mole) d'hydroxyméthyl-8-phényl -2-4H-[1]-benzopyran-4-one, 525 ml de chloroforme et 11,21 g (0,129 mole) de $MnO_2$ activé. Le tout est porté 6 heures à reflux. Le solide est filtré à chaud, repris à ébullition par 500 ml de chloroforme et les solutions chloroformiques sont réunies et concentrées sous vide. Le solide obtenu est recristallisé dans le mélange eau-acide acétique. Poids obtenu : 6,1 g (Rdt : 56,7 %).

L'hydroxyméthyl-8-phényl-2-4H-[1]-benzopyran-4-one est obtenue de la façon suivante : 13,1 g (0,041 mole) de bromométhyl-8-phényl-2-4H-[1]-benzopyran-4-one sont placés dans 104 ml de dioxanne et 104 ml d'une solution aqueuse à 5 % de bicarbonate de sodium. Le mélange est porté 2 heures à reflux, concentré sous vide et le résidu repris par 300 ml d'eau. Le précipité obtenu est essoré, séché et recristallisé dans le méthanol. Poids obtenu : 8 g (Rdt : 77 %), $PF_G$ : 175-177°C. IR : ν c = O (pyrone) : 1640 $cm^{-1}$, ν OH : 3400 $cm^{-1}$. RMN (DMSO) δ en ppm par rapport au TMS, 2 H à 5 (singulet), 1 H à 5,45 (massif inchangeable par $D_2O$) 1 H à 7 (singulet), 8 H de 7,3 à 8,3 (multiplet).

Analyse:

| | C % | H % |
|---|---|---|
| Calculée: | 76,17 | 4,80 |
| Trouvée: | 76,54 | 4,60 |

**Exemple 25**:

Acide [oxo-4-phényl-2-4H-[1]-benzopyran-8-yl]-3-propènoïque $C_{18}H_{12}O_4$ (formule 25) PM : 292,28.

On porte 8 heures à 140-145°C un mélange de 15,8 g (0,06 mole) d'[oxo-4-phényl-2-4H-[1]-benzopyran-8-yl] carboxaldéhyde, 12 ml d'anhydride acétique et 5,3 g (0,064 mole) d'acétate de sodium anhydre. Après refroidissement la masse réactionnelle est reprise par de l'eau chaude et le précipité obtenu est essoré et lavé à l'eau, on le reprend ensuite à l'ébullition par une solution de bicarbonate à 5 %, un insoluble est filtré et par acidification avec l'acide chlorhydrique on obtient un précipité que l'on recristallise dans le mélange eau-acide acétique. Poids obtenu : 9,7 g (Rdt : 52,5 %), $PF_G$ : 263-265°C. IR : ν c = O (pyrone): 1640 $cm^{-1}$, ν c = O (acide) : 1710 $cm^{-1}$. RMN (DMSO) δ en ppm par rapport au TMS. 1 H à 3,8 (massif étalé échangeable avec $D_2O$), 1 H à 6,9 (J = 15 Hz doublet), 1 H à 7,1 (singulet).

**Exemple 26**:

[oxo-4-phényl-2-4H-[1]-benzopyran-8-yl]-3-propènoate de diéthylamino- 2-éthyle, $C_{24}H_{25}NO_4$ (formule 26) PM: 391,42.

Préparé selon l'exemple 3, à partir de 3,4 g (0,011 mole) d'acide [oxo-4-phényl-2-4H-[1]-benzopyran-8-yl]-3-propénoïque, 0,77 g (0,011 mole) de potasse et 1,9 g (0,14 mole) de diéthylamino-2-chloro-1-éthane. Poids obtenu : 2,6 g (Rdt : 60,3 %), $PF_K$ 84°C (hexane). IR : ν c = O (pyrone) : 1640 $cm^{-1}$, ν c = O (ester) : 1720 $cm^{-1}$. RMN ($CDCl_3$) δ en ppm par rapport au TMS. 6 H à 1,1 (triplet), 6 H de 2,4 à 3 (multiplet) 2 H à 4,4 (triplet), 1 H à 6,8 (doublet J: 15 Hz), 1 H à 7 (singulet), 9 H de 7,2 à 8,4 (multiplet).

Chlorhydrate $C_{24}H_{26}ClNO_4$, PM = 427,92, $PF_G$ = 194-196°C (isopropanol)

Analyse pondérale:

| | C % | H % | Cl % | N % |
|---|---|---|---|---|
| Calculée: | 67,36 | 6,12 | 8,29 | 3,27 |
| Trouvée: | 67,22 | 6,07 | 8,16 | 3,21 |

**Exemple 27**:

[oxo-4-phényl-2-4H-[1]-benzopyran-8-yl]-3-propènoate de (morpholinyl- 4)-2-éthyle. $C_{24}H_{23}NO_5$ (formule 27). PM = 405,4.

Préparé selon l'exemple 3 à partir de 8,5 g (0,03 mole) d'acide [oxo-4 -phényl-2-4H-[1]-benzopyran-8-yl]-3-propénoïque, 1,92 g (0,03 mole) de potasse et 5,25 g (0,035 mole) de (chloro-2-éthyl)-4-morpholine. Poids obtenu : 9,5 g (Rdt : 78,1 %), $PF_K$ = 126°C (acétone). IR : ν c = O (pyrone) : 1640 $cm^{-1}$, ν c = O (ester) : 1720 $cm^{-1}$. RMN ($CDCl_3$) δ en ppm par rapport au TMS, 6 H de 2,5 à 3 (multiplet), 4 H de 3,7 à 4 (multiplet, 2 H à 4,5 (triplet), 1 H à 6,8 (doublet J = 15 Hz), 1 H à 7 (singulet), 9 H de 7,4 à 8,6 (multiplet).

Chlorhydrate $C_{24}H_{24}ClNO_5$, PM = 441,9, $PF_G$ = 243-5°C.

Analyse

| | C % | H % | Cl % | N % |
|---|---|---|---|---|
| Calculée: | 65,23 | 5,47 | 8,02 | 3,17 |
| Trouvée: | 65,10 | 5,38 | 8,05 | 3,08 |

**Exemple 28**:

[(méthoxy-3-phényl)-2-oxo-4-4H-[1]-benzopyran-8-yl] acétonitrile $C_{18}H_{13}NO_3$ (formule 28). PM. = 291,31.
Préparé selon l'exemple 1 à partir de 23,9 g (0,066 mole) de (bromo-méthyl)-8-(méthoxy-3-phényl)-2-4H-[1]-benzopyranone-4 et de 9,1 g de cyanure de potassium, on obtient 9,3 g avec un rendement de 46 % de produit. $PF_K$ = 170°C. IR : ν c = O = 1650 $cm^{-1}$. RMN (DMSO), 3 H à 3,9 (singulet), 2 H à 4,5 (singulet), 8 H de 6,9 à 8,3 (multiplet).

**Exemple 29**:

Acide [(méthoxy-3-phényl)-2-oxo-4-4H-[1]-benzopyran-8-yl] acétique $C_{18}H_{14}O_5$ (formule 29), PM. = 310,31;
Préparé selon l'exemple 2 à partir de 9,1 g (0,031 mole) de [(méthoxy-3-phényl)-2-oxo-4-4H-[1]-benzopyran-8-yl]-acétonitrile. Poids obtenu : 2,2 g. $PF_G$ = 238-241° (MiBK). IR : ν c = O (pyrone) = 1635 $cm^{-1}$; ν c = O (acide) = 1710 $cm^{-1}$. RMN (DMSO), 1 H à 3,4 (échangeable avec $D_2O$), 3 H à 3,9 (singulet), 2H à 4,02 (singulet), 1 H à 7,1 (singulet) 7 H de 7,1 à 8,1 (multiplet).
Analyse pondérale

| | C % | H % | O % |
|---|---|---|---|
| Calculée: | 69,67 | 4,55 | 25,78 |
| Trouvée: | 69,91 | 4,61 | |

**Exemple 30**:

(Méthoxy-6-oxo-4-phényl-2-4H-[1]-benzopyran-8-yl] acétonitrile $C_{18}H_{13}NO_3$. (formule 30). PM = 291,31.
Préparé selon l'exemple 1 à partir de 30 g (0,087 mole) de bromométhyl-8-méthoxy-6-phényl-2-4H-[1]-benzopyranone-4 et 11,9 g de cyanure de potassium. Le produit étant insoluble à chaud, on ne filtra pas à chaud, mais on refroidit, filtre à froid, lave à l'eau, sèche. Poids obtenu : 15,9 g (Rdt : 62 %), $PF_K$ = 270°C, IR : ν c = O = 1635 $cm^{-1}$. Le produit est trop peu soluble pour obtenir un spectre de RMN.

**Exemple 31**:

Acide (méthoxy-6-oxo-4-phényl-2-4H-[1]-benzopyran-8-yl] acétique $C_{18}H_{14}O_5$ (formule 31) PM = 310,31.
Préparé selon l'exemple 2 à partir de 8 g (0,027 mole) de (méthoxy-6-oxo-4-phényl-2-4H-[1]-benzopyran-8-yl) acétonitrile. Poids obtenu : 3,7 g, IR : ν c = O (pyrone) = 1630 $cm^{-1}$; ν c = O (acide) : 1720 $cm^{-1}$ ν OH = 2400 à 3500 $cm^{-1}$. La RMN montre qu'on a un mélange du produit du titre et de l'acide (hydroxy-6-oxo-4-phényl-2-4H-[1]-benzopyran-8-yl) acétique. (formule 32). Ce mélange est utilisé tel quel dans l'exemple suivant.

**Exemple 32**:

(Méthoxy-6-oxo-4-pényl-2-4H-[1]-benzopyran-8-yl) acétate de (N, N- diéthylamino)-2-éthyle $C_{24}H_{27}NO_5$. (formule 33) PM = 409,49.
Préparé selon l'exemple 3 à partir de 3,5 g du mélange d'acides de l'exemple 31 et de 1,84 g (0,013 mole) de chloro-2-N, N-diéthyléthylamine, en remplaçant l'acétone par le DMF, et en chauffant 2 h 30 à 80°C. Après évaporation, on reprend avec $CH_2Cl_2$, lave à la soude N, puis à l'eau, sèche, évapore sous vide. Poids obtenu : 1,4 g. RMN ($CDCl_3$), 6 H à 0,95 (triplet, J = 7 Hz), 6 H de 2,3 à 2,8 (multiplet), 3 H à 3,9 (singulet), 2 H à 3,96 (singulet), 2 H à 4,2 (triplet, J = 6 Hz), 1 H à 6,8 (singulet), 7 H de 7,1 à 8,1 (multiplet).

**Exemple 33:**

[(Diméthoxy-3,4-phényl)-2-oxo-4-4H[1]-benzopyran-8-yl] acétonitrile $C_{19}H_{15}NO_4$ (formule 34) PM = 321,34.

Préparé selon l'exemple 1 à partir de 30 g (0,080 mole) de (bromométhyl)-8-(diméthoxy-3,4-phényl)-2-4H[1]-benzopyranone-4 et de 11 g de cyanure de potassium. Poids obtenu : 10,5 g (Rdt : 40 %). $PF_K$ = 226°C IR : ν c = O (pyrone) = 1650 $cm^{-1}$; ν c = N = 2260 $cm^{-1}$. Le produit est trop peu soluble pour obtenir un spectre de RMN.

**Exemple 34:**

Acide [(diméthoxy-3,4-phényl)-2-oxo-4-4H-[1]-benzopyran-8-yl] acétique $C_{19}H_{16}O_6$ (formule 35) PM = 340,34.

Préparé selon l'exemple 2 à partir de 10,2 g (0,032 mole) de [(diméthoxy-3,4-phényl)-2-oxo-4-4H-[1]-benzopyran-8-yl] acétonitrile. Poids obtenu : 3,9 g, $PF_G$ = 250 - 254° (AcOH), IR : ν c = O (pyrone) = 1645 $cm^{-1}$; ν c = O (acide) = 1720 $cm^{-1}$. RMN (DMSO), 3 H à 3,83 (singulet), 3 H à 3,9 (singulet), 2 H à 4,03 (singulet), 7 H de 6,8 à 8,1 (multiplet), 1 H à 12 (échangeable avec $D_2O$).

Analyse pondérale:

| | C % | H % | O % |
|---|---|---|---|
| Calculée: | 67,05 | 4,74 | 28,21 |
| Trouvée: | 67,16 | 4,68 | |

**Exemple 35:**

[(Oxo-4-phényl-2-4H-[1]-benzopyran-8-yl) méthyl]-2-propanedioate de diéthyle $C_{23}H_{22}O_6$ (formule 36) PM = 390,24.

A une suspension de 2,3 g (0,1 mole) de sodium en billes dans 100 ml de benzène anhydre, on ajoute à température ambiante 15,6 g (0,1 mole) de propanedioate de diéthyle et on porte 5 heures à reflux, puis on laisse sous agitation 12 heures à température ambiante. On ajoute ensuite une solution de 31,5 g (0,1 mole) de bromométhyl-8-phényl-2-4H-[1]-benzopyranone-4 dans 300 ml de benzène. Le milieu est porté 7 heures à reflux. Après filtration et évaporation du solvant on isole un solide blanc que l'on recristallise. Poids obtenu : 26 g (Rdt : 66 %) $PF_G$ = 87 - 90°C (éthanol), IR ; ν c = O (ester) = 1740 $cm^{-1}$, ν c = O (pyrone) = 1650 $cm^{-1}$.

**Exemple 36:**

Acide [oxo-4-phényl-2-4H-[1]-benzopyran-8-yl]-3-propionique $C_{18}H_{14}O_4$ (formule 37). PM = 294,29.

On porte au reflux 7 heures une solution de 26 g (0,066 mole) d'[(oxo-4-phényl-2-4H-[1]-benzopyran-8-yl) méthyl]-2-propanedioate de diéthyle dans 93 ml d'acide acétique et 46 ml d'acide chorhydrique concentré. On verse le milieu réactionnel dans 800 ml d'eau, après filtration, traitement par le bicarbonate de sodium à 5 %, on isole 10,5 g (Rdt : 53 %). $PF_G$ = 201 - 202°C (acétone/éthanol). IR : ν OH (acide) = 2800-3300 $cm^{-1}$, ν c = O (acide) = 1740 $cm^{-1}$, ν c = O (pyrone) = 1640 $cm^{-1}$. RMN ($CDCl_3$) δ en ppm par rapport au TMS, 2 H à 2,6 (triplet J = 7 Hz), 2 H à 3,2 (triplet J = 7 Hz), 1 H à 7 (singulet), 8 H de 7,2 à 8,2 (multiplet), 1 H à 12,1 (échangeable avec $D_2O$).

Analyse

| | C % | H % | O % |
|---|---|---|---|
| Calculée: | 73,46 | 4,80 | 21,74 |
| Trouvée: | 73,52 | 4,55 | |

**Exemple 37:**

[(Furyl-2)-2-oxo-4-4H-[1]-benzopyran-8-yl] acétonitrile $C_{15}H_9NO_3$ (formule 38). PM = 251,22.

Préparé selon l'exemple 1 à partir de 23,4 g (0,077 mole) de bromométhyl-8-(furyl-2)-2-4H-[1]-benzopyranone-4 et 10 g (0,154 mole) de cyanure de potassium. Poids obtenu : 15,5 g (Rdt : 83 %). $PF_K$ = 195°C

(ethanol). IR : ν c = N = 2250 $cm^{-1}$, ν c = O (pyrone) : 1650 $cm^{-1}$.

**Exemple 38**:

Acide [(furyl-2)-2-oxo-4-4H[1]-benzopyran-8-yl]-acétique $C_{15}H_{10}O_5$ (formule 39). PM = 270,23.

Préparé selon l'exemple 2 à partir de 16 g (0,063 mole) de [(furyl-2)-2-oxo-4-4H-[1]-benzopyran-8-yl] acétonitrile. Poids obtenu : 8,5 g (Rdt : 49 %). $PF_G$ = 240 - 2°C. IR : ν OH = 2800 - 3200 $cm^{-1}$, ν c = O (acide) = 1720 $cm^{-1}$, ν c = O (pyrone) = 1650 $cm^{-1}$, RMN (DMSO) δ en ppm par rapport au TMS, 2 H à 4 (singulet) 1 H à 6,7 (singulet), 1 H à 6,8 à 7 (multiplet), 5 H de 7,3 à 8,1 (multiplet), 1 H à 12,6 (échangeable avec $D_2O$).

Analyse

| | C % | H % | O % |
|---|---|---|---|
| Calculée: | 66,67 | 3,73 | 29,60 |
| Trouvée: | 66,81 | 3,74 | |

**Exemple 39**:

[(Méthyl-4-phényl)-2-oxo-4-4H-[1]-benzopyran-8-yl] acétonitrile $C_{18}H_{13}NO_2$ (formule 40) · PM = 275,30.

Préparé selon l'exemple 1 à partir de 33 g (0,1 mole) de bromométhyl-8 -(méthyl-4-phényl-)-2-4H-[1]-benzopyranone-4 et 13 g (0,2 mole) de cyanure de potassium. Poids obtenu : 23,5 g (Rdt : 85,5 %). $PF_K$ =190°C IR : ν c = N = 2160 $cm^{-1}$, ν c = O (pyrone) = 1640 $cm^{-1}$.

**Exemple 40**:

Acide [(méthyl-4-phényl)-2-oxo-4-4H-[1]-benzopyran-8-yl] acétique $C_{18}H_{14}O_4$ (formule 41). PM = 294,29.

Préparé selon l'exemple 2 à partir de 23,5 g (0,085 mole) de [(méthyl-4-phényl)-2-oxo-4-4H-[1]-benzopyran-8-yl] acétonitrile. Après traitement au bicarbonate et recristallisation dans l'acide acétique on obtient 15 g (Rdt : 59 %). $PF_G$ = 250 - 252°C. IR : ν OH = 2800-3200 $cm^{-1}$, ν c = O (acide) = 1720 $cm^{-1}$, ν c = O (pyrone) = 1630 $cm^{-1}$. RMN (DMSO) δ en ppm par rapport au TMS, 3 H à 2,2 (singulet), 2 H à 4 (singulet), 1 H à 7 (singulet), 7 H de 7,2 à 8,1 (multiplet), 1 H à 12,7 (échangeable avec $D_2O$).

Analyse

| | C % | H % | O % |
|---|---|---|---|
| Calculée: | 73,46 | 4,80 | 21,74 |
| Trouvée: | 73,77 | 4,94 | |

**Exemple 41**:

[(Méthyl-4-phényl)-2-oxo-4-4H-[1]-benzopyran-8-yl] acétate de (N,N- diéthylamino)-2 éthyle. $C_{24}H_{27}NO_4$ (formule 42). PM = 393,46.

Préparé selon l'exemple 3 à partir de 16,4 g (0,0557 mole) d'acide [(méthyl-4-phényl)-2-oxo-4-4H-[1]-benzopyran-8-yl] acétique, 3,67 g (0,0557 mole) de potasse et 8,27 g (0,061 mole) de chloro-2-N,N-diéthyléthylamine. On obtient 15 g (Rdt : 70 %) d'un solide blanc. $PF_G$ = 87-89°C (diisopropyléther).

Chlorhydrate $C_{24}H_{28}ClNO_4$ . PM = 429,91.

$PF_G$ = 175-177°C (éthanol). IR :ν NH⊕ = 2400 - 2800 $cm^{-1}$, ν c = O (ester) = 1750 $cm^{-1}$, ν c = O (pyrone) = 1640 $cm^{-1}$. RMN ($CDCl_3$) δ en ppm par rapport au TMS, 6 H à 1,3 (triplet J = 7 Hz), 3 H à 2,5 (singulet), 6 H de 2,7 à 3,4 (multiplet), 2 H à 4,2 (singulet), 2 H à 4,7 (triplet, J = 6 Hz), 1 H à 6,8 (singulet), 7 H de 7,2 à 8,4 (multiplet), 1 H à 12,7 (échangeable avec $D_2O$).

Analyse

| | C % | H % | Cl % | N % | O % |
|---|---|---|---|---|---|
| Calculée: | 67,04 | 6,56 | 8,25 | 3,26 | 14,89 |
| Trouvée: | 67,30 | 6,67 | | 3,36 | |

**Exemple 42**:

[(Naphtyl-2)-2-oxo-4-4H-[1]-benzopyran-8-yl] acétonitrile $C_{21}H_{13}NO_2$ (formule 43). PM = 311,31. Préparé selon l'exemple 1 à partir de 61 g (0,167 mole) de bromométhyl -8-(naphtyl-2)-2-4H-[1]-benzopyranone -4 et 21,8 g (0,334 mole) de cyanure de potassium. On obtient 43 g (Rdt : 84 %). $PF_K$ = 189°C (éthanol), IR : ν c = N = 2260 $cm^{-1}$, ν c = O (pyrone) = 1660 $cm^{-1}$.

**Exemple 43**:

Acide [(naphtyl-2)-2-oxo-4-4H-[1]-benzopyran-8-yl] acétique $C_{21}H_{14}O_4$ (formule 44). PM = 330,32. Préparé selon l'exemple 2 à partir de 43,5 g (0,14 mole) de [(naphtyl-2)-2-oxo-4-4H-[1]-benzopyran-8-yl] acétonitrile. Par recristallisation dans l'acide acétique on obtient 29 g (Rdt : 62,7 %). $PF_G$ = 225-228°C, IR : ν OH = 2400 - 2800 $cm^{-1}$, ν c = O (acide) = 1720 $cm^{-1}$, ν c = O (pyrone) = 1640 $cm^{-1}$. RMN (DMSO) δ en ppm par rapport au TMS, 2 H à 4 (singulet), 1 H à 7,1 (singulet), 10 H de 7,2 à 8,2 (multiplet), 1 H à 12,6 (échangeable avec $D_2O$).

Analyse

| | C % | H % | O % |
|---|---|---|---|
| Calculée: | 76,35 | 4,27 | 19,38 |
| Trouvée: | 76,25 | 4,15 | |

**Exemple 44**:

[(Méthoxy-4-phényl)-2-oxo-4-4H-[1]-benzopyran-8-yl] acétonitrile $C_{18}H_{13}NO_3$ (formule 45) PM = 291,28. Préparé selon l'exemple 1 à partir de 34,5 g (0,1 mole) de bromométhyl -8-(méthoxy-4-phényl)-2-4H-[1]-benzopyranone-4 et 13 g (0,2 mole) de cyanure de potassium. On obtient 25,3 g. (Rdt : 87 %). $PF_K$ = 190°C (éthanol), IR : ν c = N = 2250 $cm^{-1}$, ν c = O (pyrone) = 1640 $cm^{-1}$.

**Exemple 45**:

Acide [(méthoxy-4-phényl)-2-oxo-4-4H-[1]-benzopyran-8-yl] acétique $C_{18}H_{14}O_5$ (formule 46). PM. = 310,29. Préparé selon l'exemple 2 à partir de 25,3 g (0,087 mole) de [(métoxy -4-phényl)-2-oxo-4-4H-[1] - benzopyran-8-yl] acétonitrile. Poids obtenu : 23,6 g (Rdt : 87,4 %), $PF_G$ = 228 - 232°C (acide acétique). IR : ν OH = 2400 - 2800 $cm^{-1}$, ν c = O (acide) = 1720 $cm^{-1}$, ν c = O (pyrone) = 1640 $cm^{-1}$. RMN (DMSO) δ en ppm par rapport au TMS, 3 H à 3,9 (singulet), 2 H à 4,1 (singulet), 8 H de 7 à 8,3 (multiplet), 1 H á 12,7 (échangeable avec $D_2O$).

Analyse

| | C % | H % | O % |
|---|---|---|---|
| Calculée: | 69,67 | 4,55 | 25,78 |
| Trouvée: | 69,64 | 4,58 | |

**Exemple 46**:

[(Méthoxy-4-phényl)-2-oxo-4-4H[1]-benzopyran-8-yl] acétate de (N,N- diéthylamino)-2-éthyle. $C_{24}H_{27}NO_5$ (formule 47). PM = 409,46.

Préparé selon l'exemple 3 à partir de 17,3 g (0,0557 mole) d'acide [(méthoxy-4-phényl)-2-oxo-4-4H-[1]-benzopyran-8-yl]-acétique, 3,67 g (0,0557 mole) de potasse et 8,27 g (0,061 mole) de chloro-2-N,N-diéty-léthylamine. Après traitement on obtient un solide que l'on recristallise dans le diisopropyléther. Poids obtenu : 16 g (Rdt : 70 %). $PF_G$ = 110°C.

Chlorhydrate $C_{24}H_{28}ClNO_5$, PM = 445,91, $PF_G$ = 161-163°C, IR : ν NH⊕ = 2400-2800 $cm^{-1}$, ν c = O (ester) = 1750 $cm^{-1}$, ν c = O (pyrone) = 1640 $cm^{-1}$. RMN ($CDCl_3$) δ en ppm par rapport au TMS, 6 H à 1,3 (triplet J = 7 Hz), 6 H de 2,8 à 3,4 (multiplet), 3 H à 3,95 (singulet), 2 H à 4,2 (singulet), 2 H à 4,65 (triplet, J = 6 Hz), 1 H à 6,8 (singulet), 7 H de 7 à 8,2 (multiplat), 1 H à 12,7 (échangeable avec $D_2O$);

Analyse

| | C % | H % | Cl % | N % | O % |
|---|---|---|---|---|---|
| Calculée: | 64,64 | 6,33 | 7,95 | 3,14 | 17,94 |
| Trouvée: | 64,50 | 6,41 | | 3,02 | |

**Exemple 47**:

(Cyclohexyl-2-oxo-4-4H-[1]-benzopyran-8-yl) acétonitrile $C_{17}H_{17}NO_2$ (formule 48). PM = 267,33.

Préparé selon l'exemple 1 à partir de 15,2 g (0,047 mole) de bromo-méthyl-8-cyclohexyl-2-4H-[1]-benzopyranone-4 et de 6,2 g (0,095 mole) de cyanure da potassium. A la fin de la réaction, on évapore sous vide, reprend à l'eau, extrait au chloroforme, sèche et évapore sous vide. Poids obtenu : 12,4 g (Rdt : 98 %), (Huile). IR : ν c ≡ N = 2240 $cm^{-1}$; ν c = O (pyrone) = 1650 $cm^{-1}$. RMN ($CDCl_3$), 11 H de 1,0 à 3,0 (multiplet), 2 H à 3,95 (singulet), 1 H à 6,2 (singulet), 2 H de 7,2 à 7,9 (multiplet), 1 H à 8,15 (doublet de doublet, $J_1$ = 8 Hz, $J_2$ = 2 Hz).

**Exemple 48**:

[Oxo-4-(phénylmétyl)-2-4H-[1]-benzopyran-8-yl] acétonitrile $C_{18}H_{13}NO_2$ · (formule 49) · PM = 275,31.

Préparé selon l'exemple 1 à partir de 47 g (0,143 mole) de bromométhyl -8-(phénylméthyl)-2-4H-[1]-benzopyranone-4 et 18,5 g (0,284 mole) de cyanure de potassium. Après filtration à chaud, on évapore sous vide et on recristallise dans un mélange éthanol-eau. Poids obtenu : 7,1 g (Rdt : 18 %), $PF_K$ = 100°C. IR : ν c = N = 2240 $cm^{-1}$; ν c = O (pyrone) = 1640 $cm^{-1}$. RMN ($CDCl_3$), 2 H à 3,83 (singulet), 2 H à 3,97 (singulet), 1 H à 6,2 (singulet), 7 H de 7,2 à 7,8 (multiplet), 1 H à 8,2 (doublet de doublet, $J_1$ = 8 Hz; $J_2$ = 2 Hz).

**Exemple 49**:

Oxo-4-phényl-3-4H-[1]-benzopyran carboxaldéhyde-8 $C_{16}H_{10}O_3$ (formule 50). PM = 250,26.

Préparé selon l'exemple 24 à partir de 88,2 g (0,28 mole) de bromo-méthyl-8-phényl-3-4H-[1]-benzopyranone-4 avec la variante suivante : après l'addition de l'acide chlorhydrique, on ne chauffe pas, mais on agite une nuit à température ambiante, puis on filtre un insoluble, on dilue le filtrat à l'eau et on filtre le précipité obtenu. Poids obtenu : 52,4 g (Rdt : 74 %). $PF_K$ = 162°C (AcOEt), IR : ν c = (pyrone) = 1650 $cm^{-1}$; ν c = O (aldéhyde) = 1700 $cm^{-1}$. RMN ($CDCl_3$), 6 H de 7,2 à 7,8 (multiplet), 1 H à 8,12 (singulet), 1 H à 8,3 (doublet de doublet, $J_1$ = 8 Hz; $J_2$ = 2 Hz), 1 H à 8,6 (doublet de doublet, $J_1$ = 8 Hz; $J_2$ = 2 Hz), 1 H à 10,7 (singulet).

**Exemple 50**:

Acide (cyclohexyl-2-oxo-4-4H-[1]-benzopyran-8-yl] acétique $C_{17}H_{18}O_4$. (formule 51). PM = 286,33.

Préparé selon l'exemple 2 à partir de 12,4 g (0,046 mole) de (cyclo-hexyl-2-oxo-4-4H-[1]-benzopyran-8-yl) acétonitrile. Aprés dilution à l'eau, on extrait au chloroforme, lave à l'eau, évapore sous vide, dissout dans une solution aqueuse de bicarbonate de sodium à 5 %, filtre un insoluble, refroidit, acidifie, filtre et recristallise dans le toluène. Poids obtenu : 3,4 g (Rdt : 25 %). $PF_G$ = 180-182°C. IR : ν c = O (pyrone) = 1645 $cm^{-1}$, ν c = O (acide) = 1700 $cm^{-1}$. RMN (DMSO), 11 H de 1,0 à 3,0 (multiplet), 2 H à 3,87 (singulet), 1 H à 6,2 (singulet), 1 H à 7,4 (triplet, J = 8 Hz), 1 H à 7,72 (doublet de doublet, $J_1$ = 8 Hz, $J_2$ = 2 Hz), 1 H à 7,97 (doublet de doublet, $J_1$ = 8 Hz, $J_2$ = 2 Hz).

Analyse pondérale

| | C % | H % | O % |
|---|---|---|---|
| Calculée: | 71,31 | 6,34 | 22,35 |
| Trouvée: | 71,48 | 6,35 | |

**Exemple 51**:

Acide (oxo-4-phényl-3-4H-[1]-benzopyran-8-yl) acétique $C_{17}H_{12}O_4$. (formule 52). PM = 280,28.

A un mélange de 4,8 g (0,1 mole) d'une suspension d'hydrure de sodium à 50 % dans l'huile, et de 120 ml de dioxane anhydre, on ajoute goutte à goutte une solution de 33,1 g (0,1 mole) de diméthylaminométhylène disphosphonate de tétraéthyle (C.R. DEGENHARDT, Synth. Commun 1982 12 415) dans 120 ml de dioxanne. On agite 1 h à 25°C puis on ajoute goutte à goutte une solution de 25 g (0,1 mole) d'oxo-4-phényl-3-4H-[1]-benzopyrancarboxaldéhyde-8 dans 120 ml de dioxanne. On chauffe 1 H à 50°C, puis on évapore sous vide, reprend à l'eau, extrait au chloroforme, et évapore sous vide. Le résidu est repris avec 1,5 l d'acide chlorhydrique concentré et chauffé 30 minutes à reflux. On refroidit, dilue à l'eau glacée, extrait au chloroforme, lave à l'eau, sèche et évapore sous vide. Le résidu est repris avec 300 ml d'une solution aqueuse de bicarbonate de sodium à 5 % à reflux, filtré, le filtrat est refroid et acidifié (HCl 6 N); Le précipité pâteux obtenu est extrait au chloroforme, séché et évaporé sous vide. Le résidu est purifié par chromatographie ($SiO_2$, $C_6H_6$-AcOH-MeOH-45 : 8 : 8) puis recristallisé dans le toluène. Poids obtenu : 1,1 g. $PF_G$ = 137-139°C. IR : ν c = O (pyrone) = 1640 $cm^{-1}$; ν c = O (acide) = 1730 $cm^{-1}$. RMN ($CDCl_3$), 2 H a 3,97 (singulet), 7 H de 7,1 a 7,8 (multiplet), 1 H à 8,05 (singulet), 1 H à 8,3 (doublet de doublet, $J_1$ = 8 Hz, $J_2$ = 2 Hz), 1 H à 8,8 (échangeable avec $D_2O$).

Analyse pondérale

| | C % | H % | O % |
|---|---|---|---|
| Calculée: | 72,85 | 4,32 | 22,83 |
| Trouvée: | 72,83 | 4,50 | |

**Exemple 52**:

Acide [oxo-4-(phénylméthyl)-2-4H-[1]-benzopyran-8-yl] acétique $C_{18}H_{14}O_4$ (formule 53). PM = 294,31.

Préparé selon l'exemple 2 à partir de 7 g (0,025 mole d'[oxo-4-(phénylméthyl)-2-4H-[1]-benzopyran-8-yl] acétonitrile. Poids obtenu : 2,7 g (Rdt : 36 %). $PF_G$ = 143-145°C (toluène). IR : ν c = O (pyrone) = 1640 $cm^{-1}$; ν c = O (acide) = 1720 $cm^{-1}$. RMN ($CDCl_3$), 2 H à 3,87 (singulet), 2 H à 3,93 (singulet), 1 H à 6,27 (singulet), 9 H de 7,0 à 8,3 (multiplet).

Analyse pondérale

| | C % | H % | O % |
|---|---|---|---|
| Calculée: | 73,46 | 4,79 | 21,75 |
| Trouvée: | 73,40 | 4,82 | |

**Exemple 53**:

(Diphényl-2,3-oxo-4-4H-[1]-benzopyran-8-yl) acétonitrile $C_{23}H_{15}NO_2$ (formule 54). PM = 337,38.

Préparé selon l'exemple 1 à partir de 22,5 g (0,057 mole) de bromo-méthyl-8-diphényl-2,3-4H-[1]-benzopyranone-4 et de 7,6 g de cyanure de potassium. Poids obtenu : 7,1 g (Rdt : 36 %). $PF_K$ = 185°C. IR : ν c = O (pyrone) = 1630 $cm^{-1}$. RMN ($CDCl_3$) 2 H à 4,0 (singulet), 12 H de 7,0 à 8,0 (multiplet), 1 H à 8,3 (doublet de doublet $J_1$ = 8 Hz, $J_2$ = 2 Hz).

**Exemple 54**:

Acide (diphényl-2,3-oxo-4-4H-[1]-bénzopyran-8-yl) acétique $C_{23}H_{16}O_4$ (formule 55). PM = 356,38.

Préparé selon l'exemple 2 à partir de 7,1 g (0,021 mole) de (diphényl-2,3-oxo-4-4H-[1]-benzopyran-8-yl) acétonitrile. Poids obtenu : 2,4 g. $PF_G$ = 220 - 224° C. IR : ν c = O (acide) = 1730 $cm^{-1}$, ν c = O (pyrone) = 1630 $cm^{-1}$, ν OH = 2900 à 3600 $cm^{-1}$. RMN (DMSO), 1 H à 3,5 (échangeable avac $D_2O$), 2 H à 4,0 (singulet), 13 H de 7,0 à 8,3 (multiplet).

Analyse pondérale

| | C % | H % | O % |
|---|---|---|---|
| Calculée: | 77,51 | 4,53 | 17,96 |
| Trouvée: | 77,29 | 4,51 | |

**Exemple 55**:

(Méthyl-2-oxo-4-4H-[1]-benzopyran-8-yl) acétonitrile $C_{12}H_9NO_2$ (formule 56). PM = 199,21.

Préparé selon l'exemple 1 à partir de 8,8 g (0,034 mole) de bromo-méthyl-8-méthyl-2-4H-[1]-benzopyranone-4. Poids obtenu : 5,1 g. IR : ν c = N = 2250 $cm^{-1}$, ν c = O (pyrone) = 1650 $cm^{-1}$. RMN ($CDCl_3$), 3 H à 2,4 (singulet), 2 H à 3,97 (singulet), 1 H à 6,2 (singulet), 1 H à 7,4 (triplet, J = 8 hz), 1 H à 7,7 (doublet de doublet, $J_1$ = 8 Hz, $J_2$ = 2 Hz) 1 H à 8,2 (doublet de doublet, $J_1$ = 8 Hz, $J_2$ = 2 Hz).

**Exemple 56**:

Acide (méthyl-2-oxo-4-4H-[1]-benzopyran-8-yl) acétique $C_{12}H_{10}O_4$ (formule 57). PM = 218,21.

Préparé selon l'exemple 2 à partir de 5 g (0,025 mole) de (méthyl-2-oxo-4-4H-[1]-benzopyran-8-yl) acétonitrile. Après traitement au bicarbonate et acidification, le produit est purifié par chromatographie ($SiO_2$, $C_6H_6$-$CH_3$-COOH-MeOH, 45 : 8: 8) puis recristallisé dans un mélange eau-acide acétique. Poids obtenu : 0,6 g. $PF_G$ = 230-233° C. IR ν c = O (acide) = 1720 $cm^{-1}$, ν c = O (pyrone) = 1635 $cm^{-1}$, ν c = O (pyrone) = 2300 à 3300 $cm^{-1}$. RMN (DMSO), 3 H à 2,36 (singulet), 1 H à 3,4 (échangeable avec $D_2O$), 2 H à 3,83 (singulet), 1 H à 6,25 (singulet), 1 H à 7,4 (triplet, J = 8 Hz), 1 H à 7,7 (doublet de doublet, $J_1$ = 8 Hz, $J_2$ = 2 Hz), 1 H à 7,9 (doublet de doublet, $J_1$ = 8 Hz, $J_2$ = 2 Hz).

Analyse pondérale

| | C % | H % | O % |
|---|---|---|---|
| Calculée: | 66,05 | 4,62 | 29,33 |
| Trouvée: | 66,14 | 4,75 | |

**Revendications** pour les Etats contractants BE CH DE FR GB IT LI LU NL SE

1. Acides [oxo-4-4H-[1]-benzopyran-8-Yl] alcanoïques caractérisés par la formule

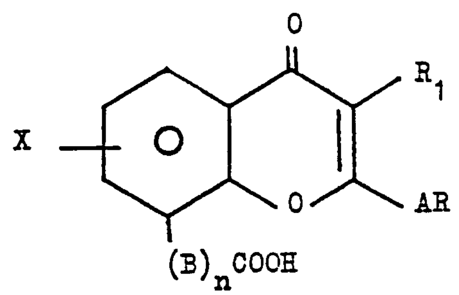

dans laquelle AR est un radical méthyle, phényle, méthylphényle, méthoxyphényle, diméthoxyphényléthényle, furyle, naphtyle, cyclohexyle, ou benzyle, B = $CH_2$; $R_1$ est l'hydrogène, X est l'hydrogène, n = 1, ainsi que leurs sels de métaux alcalins.

2. Acides [oxo-4-4H-[1]-benzopyran-8-Yl] alcanoïques caractérisés par la formule

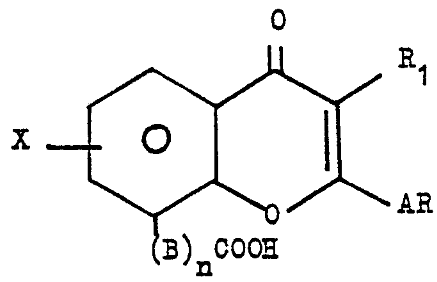

dans laquelle AR est un radical phényle, $R_1$ est l'hydrogène, X est un radical méthyle, n = 1, B = $CH_2$, ou

$CH_3-CH$,

ainsi que leurs sels de métaux alcalins.

3. Acides [oxo-4-4H-[1]-benzopyran-8-Yl] alcanoïques caractérisés par la formule

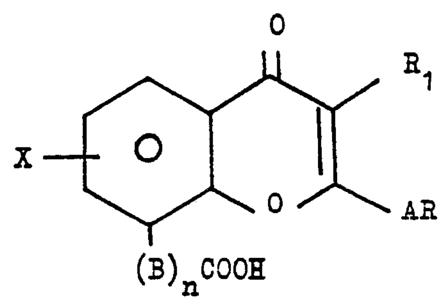

dans laquelle AR est un radical phényle, $R_1$ est l'hydrogène, B = $CH_2$, n = 1, X = le radical hydroxy ou méthoxy, ainsi qué leurs sels de métaux alcalins.

4. Acides [oxo-4-4H-[1]-benzopyran-8-Yl] alcanoïques, caractérisés par la formule

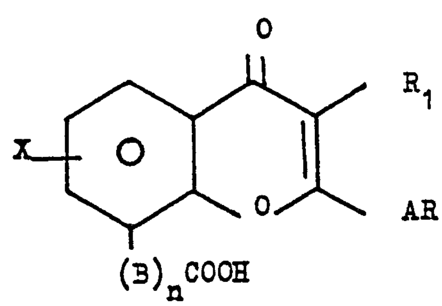

dans laquelle AR est un radical phényle, B =

$CH_3-CH$

ou $CH_2$-$CH_2$ ou CH = CH; $R_1$ et X sont l'hydrogène, n = 1, ainsi que leurs sels de métaux alcalins.

5. Acides [oxo-4-4H-[1]-benzopyran-8-Yl] alcanoïques caractérisés par la formule

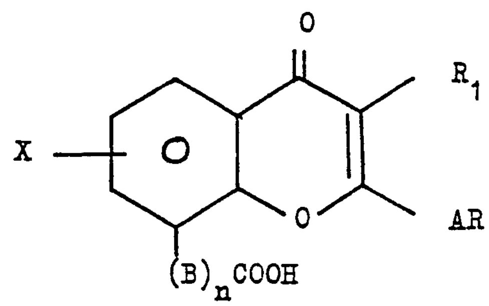

dans laquelle AR est un radical phényle ou hydrogène, n = 1, $R_1$ = un radical phényle, X est l'hydrogène, B = $CH_2$, ainsi que leurs sels de métaux alcalins.

6. Esters et aminoesters d'un composé selon la revéndication 1, caractérisés par la formule

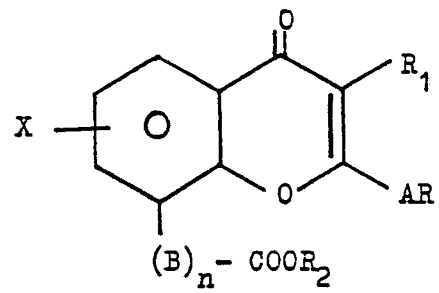

dans laquelle AR est un radical phényle, $R_1$ = hydrogène, X est l'hydrogène, n = 1, B = $CH_2$, $R_2$ est un radical méthyle, éthyle, hydroxyéthyle, diéthylaminoéthyle, diméthylaminopropyle ou morpholinoéthyle.

7. Esters et aminoesters d'un composé selon la revendication 1 de formule

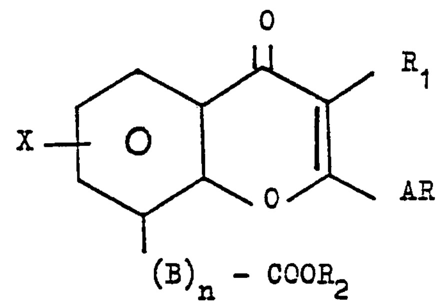

dans laquelle AR est un radical phénylé, $R_1$ = hydrogène, X est l'hydrogène, n = 1, B = $CH_2$, $R_2$ est un radical méthyle ou diéthylaminoéthyle.

8. Esters et aminoesters d'un composé selon la revendication 2, de formule

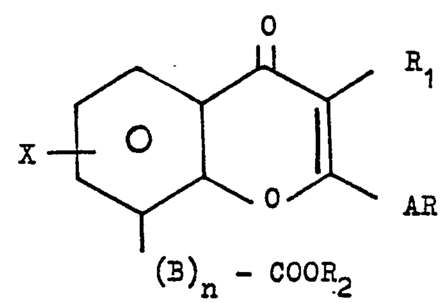

dans laquelle AR est un radical phényle, $R_1$ est l'hydrogène, n = 1, B = $CH_2$, X = radical méthyle et $R_2$ est un radical méthyle ou diéthylaminoéthyle.

9. Aminoester d'un composé selon la revendication 2, de formule

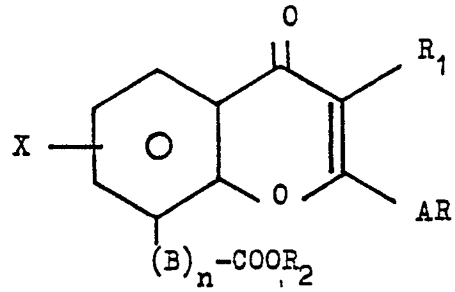

dans laquelle AR est un radical phényle, $R_1$ est l'hydrogène, B =

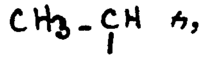

n = 1, X = hydrogène, et $R_2$ est le radical diéthylaminoéthyle.

10. Aminoesters d'un composé selon la revendication 4, de formule

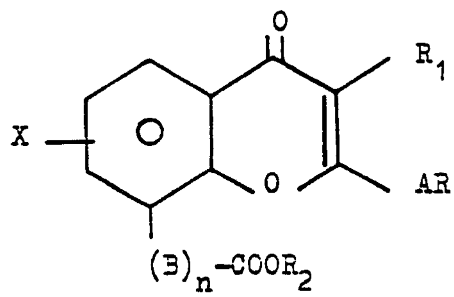

dans laquelle AR est un radical phényle, $R_1$ et X sont l'hydrogène, B = CH = CH, n = 1, $R_2$ est un radical diéthylaminoéthyle ou morpholinoéthyle.

11. Aminoester d'un composé selon la revendication 3, de formule

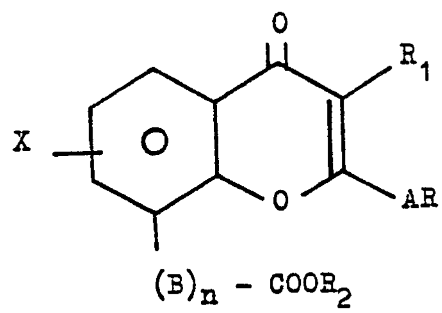

dans laquelle AR est un radical phényle, $R_1$ = hydrogène, X un radical méthoxy, B = $CH_2$, n = 1 et $R_2$ est le radical diéthylaminoéthyle.

12. Aminoesters d'un composé selon la revendication 1 de formule

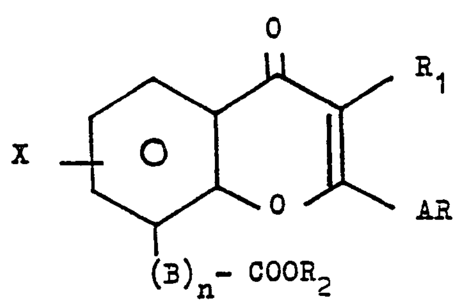

dans laquelle AR est un radical méthylphényle ou méthoxyphényle, $R_1$ et X = hydrogène, B = $CH_2$, n = 1 et $R_2$ est la radical diéthylaminoéthyle.

13. Amide d'un composé sélon la revendication 1, de formule

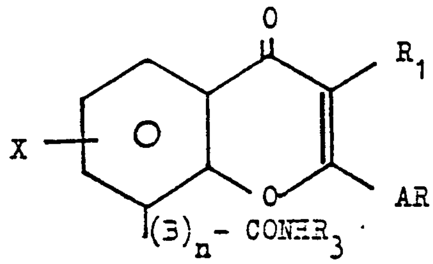

dans laquelle AR est un radical phényle, $R_1$ et X sont l'hydrogène, B = $CH_2$, n = 1, $R_3$ est le radical diéthylaminoéthyle.

14. Procédé de préparation des acides selon une quelconque des revendications 1 à 5, caractérisé en ce que quand B = $CH_2$ on hydrolyse les nitriles correspondants, les dits nitriles étant obtenus par traitement du bromo-méthyl-8-bénzopyranone-4 correspondant avec un cyanure alcalin; quand B : CH = CH on met en réaction le bromométhyl-8-benzopyranone-4 correspondant avec l'hexaméthylènetétramine, puis procède à la condensation de l'aldéhyde obtenu avec l'anhydride acétique en présence d'acétate de sodium ; quand B est un radical $CH_2$-$CH_2$ ou

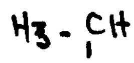

on hydrolyse le malonate correspondant.

15. Nitriles utilisables dans la préparation des acides selon la revendication 1, représentés par la formule

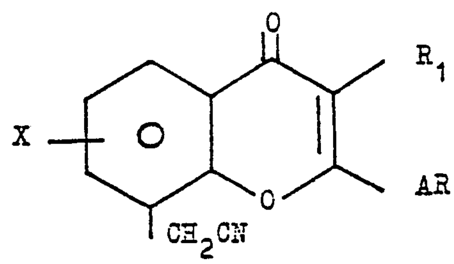

dans laquelle AR est un radical méthyle, phényle, méthylphényle, méthoxyphényle, diméthoxyphényle, benzyle, cyclohexyle, thényle, furyle, naphtyle, $R_1$ et X sont l'hydrogène.

16. Nitriles utilisables dans la préparation des acides selon la revendication 2 ou 3 représentés par la formule

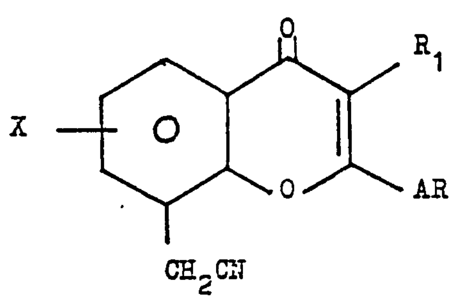

dans laquelle AR est le radical phényle, $R_1$ est l'hydrogène, X est un radical méthyle ou méthoxy.

17. Nitrile utilisable dans la préparation d'un composé selon la revendication 5, représenté par la formule

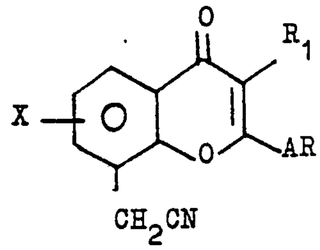

dans laquelle AR est le radical phényle, $R_1$ est le radical phényle et X est l'hydrogène.

18. Malonate utilisable dans la préparation des acides selon la revendication 4, représenté par la formule

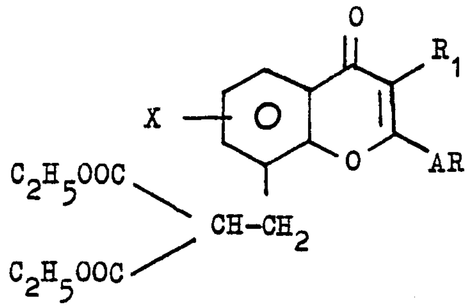

dans laquelle AR est un radical phényle, $R_1$ l'hydrogène, X représente l'hydrogène.

19. Malonates utilisables dans la préparation d'un acide selon la revendication 2 ou 4, représentés par la formule

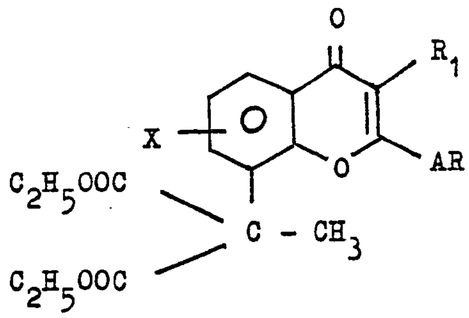

dans laquelle AR est un radical phényle, $R_1$ l'hydrogène, X le radical méthyle.

20. [oxo-4 phényl-2-4H-[1]-benzopyran-8-Yl] carboxaldéhyde, et [oxo-4 phényl-3-4H-[1] -benzopyran-8-Yl] carboxaldéhyde en tant que produits intermédiaires utilisables dans la préparation d'acides.

21. Médicament contenant comme principe actif un acide ou un sel de métal alcalin selon la revendication 1.

22. Médicament contenant comme principe actif un acide ou un sel de métal alcalin selon la revendication 2.

23. Médicament contenant comme principe actif un acide ou un sel de métel alcalin selon la revendication 3.

24. Médicament contenant comme principe actif un acide ou un sel de métal alcalin selon la revendication 4.

25. Médicament contenant comme principe actif un acide ou un sel de métal alcalin selon la revendication 5.

26. Médicament contenant comme principe actif un ester ou aminoester selon la revendication 6.

27. Médicament contenant comme principe actif un ester ou aminoester selon la revendication 7.

28. Médicament contenant comme principe actif un ester ou aminoester selon la revendication 8.

29. Médicament contenant comme principe actif un aminoester selon la revendication 9.

30. Médicament contenant comme principe actif un aminoester selon la revendication 10.

31. Médicament contenant comme principe actif un aminoester selon la revendication 11.

32. Médicament contenant comme principe actif un aminoester selon la revendication 12.

33. Médicament contenant comme principe actif un amide selon la revendication 13.

**Revendications** pour l'Etat contractant AT

1. Procédé de préparation des acides [oxo-4-4H-[1]-benzopyran-8-yl] alcanoïques de formule

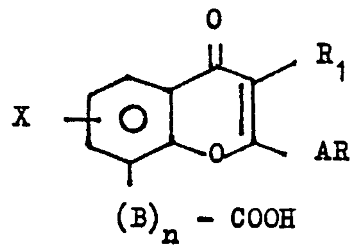

dans laquelle AR est un radical méthyle, phényle, méthylphényle, méthoxyphényle, diméthoxyphényle, thényle, furyle, naphtyle, cyclohexyle ou benzyle, B = $CH_2$; $R_1$ est l'hydrogène, X est l'hydrogène, n = 1, caractérisé en ce qu'on hydrolyse les nitriles correspondants.

2. Procédé de préparation des acides [oxo-4-4H-[1]-benzopyran-8-yl] alcanoïques de formule

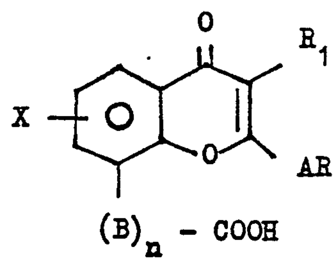

dans laquelle AR est un radical phényle, $R_1$ est l'hydrogène, X est un radical méthyle, n = 1, B = $CH_2$, $CH_2$-$CH_2$ ou

$CH_3$-CH,

caractérisé en ce quand B = $CH_2$ on hydrolyse le nitrile correspondant; quand B =

$CH_3$-CH

on réalise l'introduction du radical ramifié par transformation de l'acide en ester puis en malonate qui est alcoylé puis hydrolysé en acide correspondant; quand B = $CH_2$-$CH_2$ on fait réagir une bromométhyl-8 benzopyranone-4 avec le malonate de diéthyle en présence d'un agent basique, puis le malonate substitué obtenu est hydrolysé en acide correspondant.

3. Procédé de préparation des acides [oxo-4-4H-[1]-benzopyran-8-yl] alcanoïques de formule

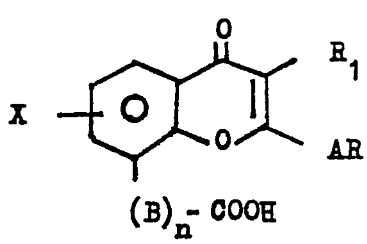

dans laquelle AR est un radical phényle, $R_1$ est l'hydrogène, B = $CH_2$, n = 1, X = le radical hydroxy ou méthoxy, caractérisé en ce qu'on hydrolyse les nitriles correspondants.

4. Procédé de préparation des acides [oxo-4-4H-[1]-benzopyran-8-yl] alcanoïques de formule

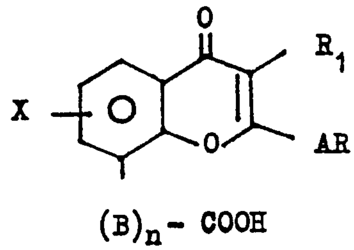

dans laquelle AR est un radical phényle, B =

$$CH_3-CH$$

ou CH = CH; $R_1$ et X sont l'hydrogène, n = 1, caractérisé en ce quand B =

$$CH_3-CH$$

on réalise l'introduction du radical ramifié par transformation de l'acide en ester puis en malonate qui est alcoylé puis hydrolysé en l'acide attendu, et quand B = CH = CH on met en réaction un bromométhyl-8 benzopyranone-4 avec l'hexaméthylènetétramine, puis on procède à la condensation da l'aldéhyda obtanu avec l'anhydride acétique en présence d'acétate da sodium.

5. Procédé de préparation des acides [oxo-4-4H-[1]-benzopyran-8-yl] alcanoïques de formule

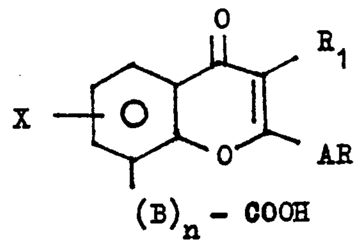

dans laquelle AR est un radical phényle ou hydrogène, n = 1, $R_1$ = un radical phényle, X est l'hydrogène, B = $CH_2$ caractérisé en ce qu'on hydrolyse les nitriles correspondants.

6. Procédé de préparation des nitriles utilisables dans la préparation des acides selon la revendication 1, représentés par la formule

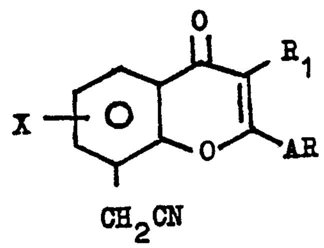

dans laquelle AR est un radical méthyle, phényle, méthylphényle, méthoxyphényle, diméthoxyphényle, benzyle, cyclohexyle, thényle, furyle, naphtyle, $R_1$ et X sont l'hydrogène, caractérisé en ce qu'on traite un bromométhyl-8 benzopyranone-4 de formule

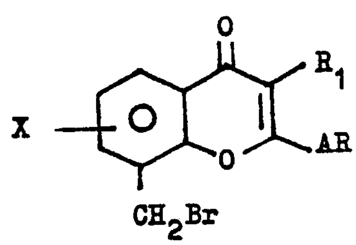

dans laquelle AR, $R_1$ et X ont les mêmes significations que ci-dessus, avec un cyanure alcalin.

7. Procédé de préparation des nitriles utilisables dans la préparation des acides selon la revendication 2 ou 3 représentés par la formule

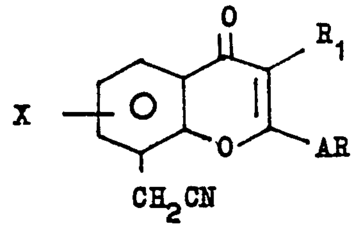

dans laquelle AR est le radical phényle, $R_1$ est l'hydrogène, X est un radical méthyle ou méthoxy, caractérisé en ce qu'on traite un bromométhyl-8 benzopyranone-4 de formule

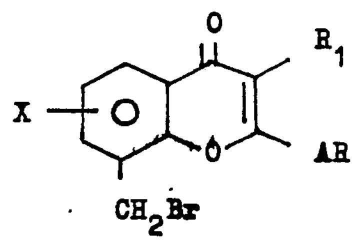

dans laquelle AR, $R_1$ et X ont les mêmes significations que ci-dessus, avec un cyanure alcalin.

8. Procédé de préparation des nitriles utilisables dans la préparation d'un composé selon la revendication 5, représenté par la formule

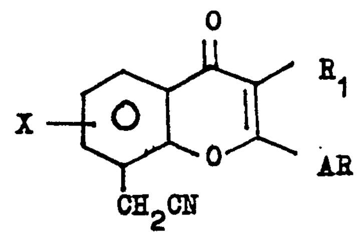

dans laquelle AR est le radical phényle, $R_1$ est le radical phényle et X est l'hydrogène, caractérisé en ce qu'on traite le bromométhyl-8 benzopyranone-4 de formule

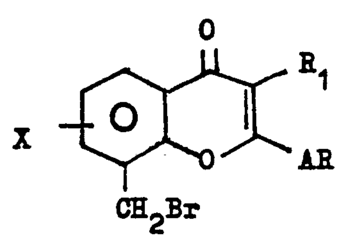

dans laquelle AR, $R_1$ et X ont les mêmes significations que-ci-dessus, avec un cyanure alcalin.

9. Procédé de préparation d'un malonate utilisable dans la préparation des acides selon la revendication 4, représenté par la formule

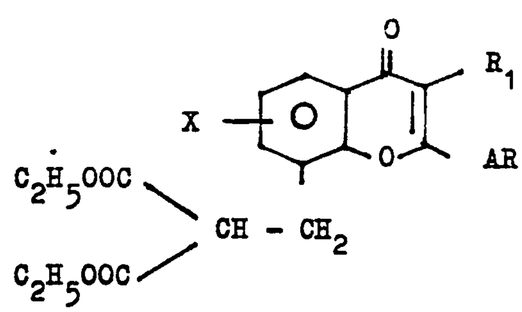

dans laquelle AR est un radical phényle, $R_1$ est l'hydrogène, X représente l'hydrogène, caractérisé par la transformation d'un acide en ester puis en malonate.

10. Procédé de préparation de malonates utilisables dans la préparation d'un acide selon la revendication 2

ou 4, représentés par la formule

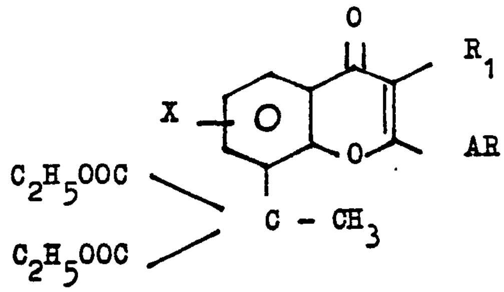

dans laqualle AR est un radical phényle, $R_1$ est l'hydrogène, X est le radical, méthyle caractérisé par la transformation d'un acide en ester puis en malonate.

11. Procédé de préparation des [oxo-4 phényl-2-4H-[1] -benzopyran-8-yl] carboxaldéhyde et [oxo-4 phényl-3-4H-[1]-benzopyran-8-yl] carboxaldéhyde produits intermédiaires utilisables dans la préparation des acides, caractérisé par la condensation d'un bromométhyl-8 benzopyranone-4 avec l'hexaméthylènetétramine.

12. Procédé de préparation du N-[N,N'-diéthylamino-2 éthyl] [oxo-4-phényl-2-4H-[1]-benzopyran-8-yl] acétamide, caractérisé par la condensation de l'[oxo-4-phényl-2-4H-[1]-benzopyran-8-yl] acétate d'éthyle avec la diéthylamino-2 éthylamine.

13. Procédé de préparation des sels de métaux alcalins des acides selon les revendications 1 à 5, caractérisé par la neutralisation des acides précédents.

14. Procede de préparation des esters et aminoesters d'un composé selon la revendication 1, de formule

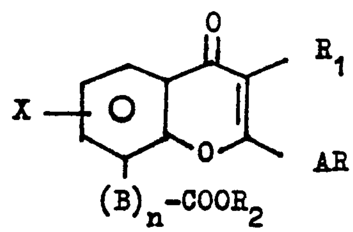

dans laquelle AR est un radical phényle, $R_1$ est l'hydrogène, X est l'hydrogène, n = 1, B = $CH_2$, $R_2$ est un radical méthyle, éthyle, hydroxyéthyle, diéthylaminoéthyle, diméthylaminopropyle ou morpholinoéthyle, caractérisé en ce que les esters sont obtenus par estérification par un alcool des acides précédents, et les aminoesters par condensation des dits acides avec un chloroalkylaminodialkyle selon les significations du substituant $R_2$.

15. Procédé de préparation des esters et aminoasters d'un composé selon la revendication 1 de formule

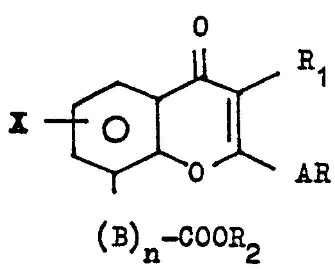

dans laquelle AR est un radical thényle, $R_1$ est l'hydrogène, X est l'hydrogène, n = 1, B = $CH_2$, $R_2$ est un radical méthyle ou diéthylaminoéthyle, caractérisé par l'estérification de l'acide par le méthanol, et dans le cas de l'aminoester par condensation de l'acide avec le chloroéthylaminodiéthyle.

16. Procédé de préparation des esters et aminoesters d'un composé selon la revendication 2, de formule

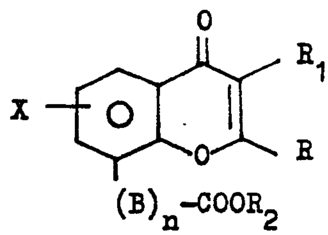

dans laquelle AR est un radical phényle, $R_1$ est l'hydrogène, $n = 1$, $B = CH_2$, X est un radical méthyle et $R_2$ est un radical méthyle ou diéthylaminoéthyle, caractérisé par l'estérification de l'acide par le méthanol et dans le cas de l'aminoester par la condensation de l'acide avec le chloroéthylaminodiéthyle.

17. Procédé de préparation d'un aminoester d'un composé selon la revendication 2, de formule

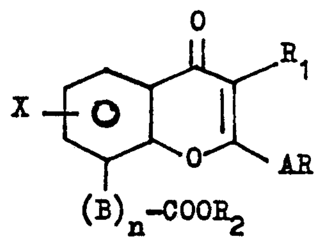

dans laquelle AR est un radical phényle, $R_1$ est l'hydrogène, B =

$CH_3-CH$,

$n = 1$, X est l'hydrogène et $R_2$ est le radical diéthylaminoéthyle, caractérisé par la condensation de l'acide avec le chloroéthylaminodiéthyle.

18. Procédé de préparation d'aminoesters d'un composé selon la revendication 4, de formule

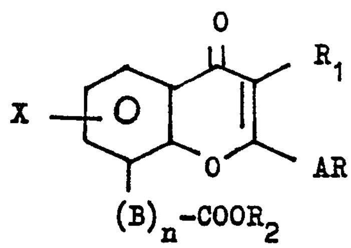

dans laquelle AR est un radical phényle, $R_1$ et X sont l'hydrogène, $B = CH = CH$, $n = 1$, $R_2$ est un radical diéthylaminoéthyle ou morpholinoéthyle, caractérisé en ce que l'acide est condensé avec le chloroéthylaminodiéthyle ou la chloroéthylmorpholine.

19. Procédé de préparation d'un composé de formule

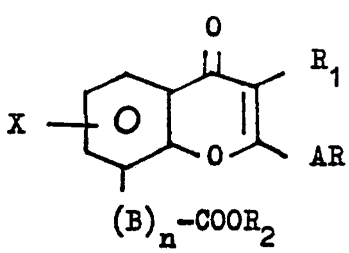

dans laquelle AR est un radical phényle, $R_1$ = hydrogène, X un radical méthoxy, $B = CH_2$, $n = 1$ et $_2R$ est le radical diéthylaminoéthyle, caractérisé en ce que l'acide est condensé avec le chloroéthylaminodiéthyle.

20. Procédé de préparation de composés de formule

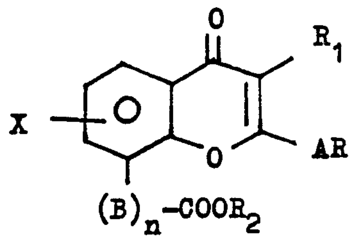

dans laquelle AR est un radical méthylphényle ou méthoxyphényle, $R_1$ et X sont l'hydrogène, B = $CH_2$, n = 1 et $R_2$ est le radical diéthylaminoéthyle, caractérisé en ce que l'acide est condensé avec le chloroéthylaminodiéthyle.

**Patentansprüche** für die Vertragsstaaten BE, CH, DE, FR, GB, IT, LI, LU, NL, SE

1. (4-Oxo-4H-[1]-benzopyran-8-yl]-alkansäuren, gekennzeichnet durch die Formel

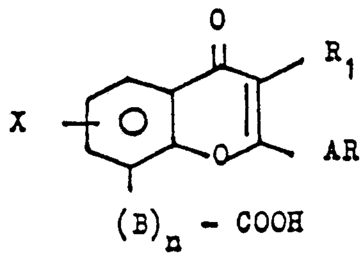

worin AR ein Methyl-, Phenyl-, Methylphenyl-, Methoxyphenyl-, Dimethoxyphenyl-, Thenyl-, Furyl-, Naphthyl-, Cyclohexyl- oder Benzylrest ist, B = $CH_2$, $R_1$ Wasserstoff ist, X Wasserstoff ist und n = 1, sowie deren Alkalimetallsalze.

2. (4-Oxo-4H-[1]-benzopyran-8-yl)-alkansäuren, gekennzeichnet durch die Formel

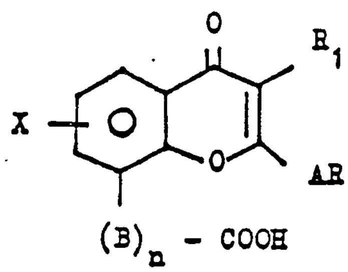

worin R ein Phenylrest ist, $R_1$ Wasserstoff ist, X ein Methylrest ist, n, 1, B = $CH_2$ oder

$CH_3-CH$,

sowie deren Alkalimetallsalze.

3. (4-Oxo-4H-[1]-benzopyran-8-yl)-alkansäuren, gekennzeichnet durch die Formel

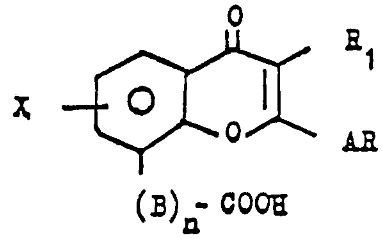

worin AR ein Phenylrest ist, $R_1$ Wasserstoff ist, B = $CH_2$, n = 1 und X = der Hydroxyl- oder Methoxyrest, sowie deren Alkalimetallsalze.

4. (4-Oxo-4H-[1]-benzopyran-8-yl)-alkansäuren, gekennzeichnet durch die Formel

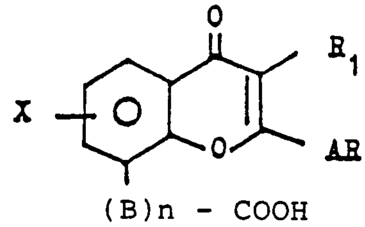

worin AR ein Phenylrest ist, B =

$$CH_3-\underset{|}{C}H$$

oder $CH_2$-$CH_2$ oder CH=CH, $R_1$ und X Wasserstoff sind und n = 1, sowie deren Alkalimetallsalze.

5. (4-Oxo-4H- [1]-benzopyran-8-yl)-alkansäuren, gekennzeichnet durch die Formel

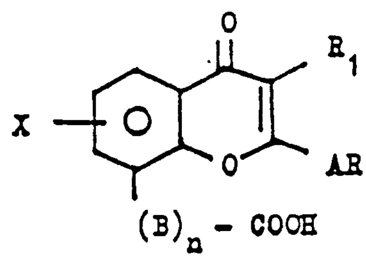

worin AR ein Phenylrest oder Wasserstoff ist, n = 1, $R_1$ = ein Phenylrest, X Wasserstoff ist und B = $CH_2$, sowie deren Alkalimetallsalze.

6. Ester und aminoester einer Verbindung nach Anspruch 1, gekennzeichnet durch die Formel

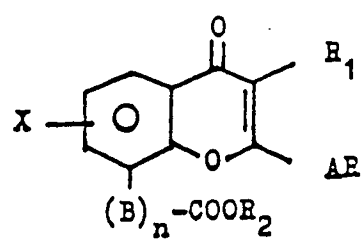

worin AR ein Phenylrest ist, $R_1$ = Wasserstoff, X Wasserstoff ist, n = 1, B = $CH_2$ und R ein Methyl-, Ethyl-, Hydroxyethyl-, Diethylaminoethyl-, Dimethylaminopropyl- oder Morpholinoethylrest ist.

7. Ester und Aminoester einer Verbindung nach Anspruch 1 der Formel

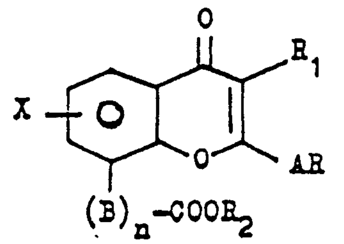

worin AR ein Thenylrest ist, $R_1$ = Wasserstoff, X Wasserstoff ist, n = 1, B = $CH_2$ und $R_2$ ein Methyl- oder Diethylaminoethylrest ist.

8. Ester und Aminoester einer Verbindung nach Anspruch 2 der Formel

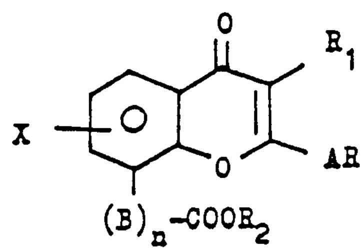

worin AR ein Phenylrest ist, $R_1$ Wasserstoff ist, n = 1, B = $CH_2$, X = der Methylrest und $R_2$ ein Methyl- oder Diethylaminoethylrest ist.

9. Aminoester einer Verbindung nach Anspruch 2 der Formel

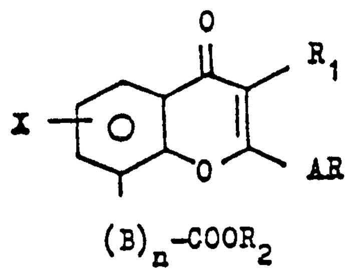

worin AR ein Phenylrest ist, $R_1$ Wasserstoff ist, B =

$$CH_3-CH,$$

n = 1, X = Wasserstoff und $R_2$ der Diethylaminoethylrest ist.

10. Aminoester einer Verbindung nach Anspruch 4 der Formel

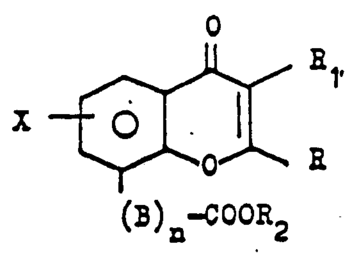

worin AR ein Phenylrest ist, $R_1$ und X Wasserstoffatome sind, B = CH=CH, n = 1 und $R_2$ ein Diethylaminoethyl- oder Morpholinoethylrest ist.

11. Aminoester einer Verbindung nach Anspruch 3 der Formel

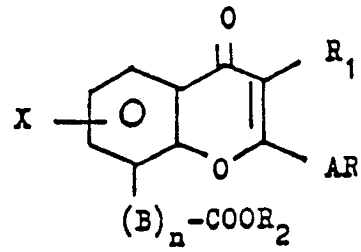

worin AR ein Phenylrest ist, $R_1$ = Wasserstoff, X ein Methoxyrest ist, B = $CH_2$, n = 1 und $R_2$ der Diethylaminoethylrest ist.

12. Aminoester einer Verbindung nach Anspruch 1 der Formel

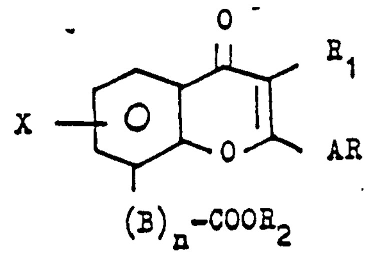

worin AR ein Methylphenyl- oder Methoxyphenylrest ist, $R_1$ und X = Wasserstoff, B = $CH_2$, n = 1 und $R_2$ ein Diethylaminoethylrest ist.

13. Amid einer Verbindung nach Anspruch 1 der Formel

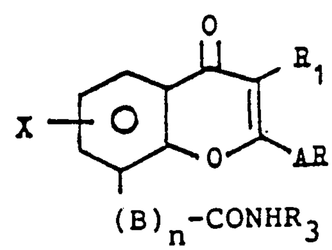

worin AR ein Phenylrest ist, $R_1$ und X Wasserstoff sind, B = $CH_2$, n = 1 und $R_3$ der Diethylaminoethylrest ist.

14. Verfahren zur Herstellung von Säuren nach einem der Ansprüche 1 bis 5, dadurch gekennzeichnet, daß man, wenn B = $CH_2$, die entsprechenden Nitrile hydrolysiert, wobei diese Nitrile durch Behandlung des entsprechenden 8-Brommethylbenzopyranon-4 mit einem Alkalicyanid erhalten werden, wenn B = CH=CH, das ensprechende 8-Brommethylbenzopyranon-4 mit Hexamethylentetramin in Reaktion bringt und dann den erhaltenen Aldehyd mit Acetanhydrid in Gegenwart von Natriumacetat der Kondensation unterzieht und, wenn B ein $CH_2$-$CH_2$- oder

$$CH_3-\underset{|}{C}H$$

-Rest ist, das entsprechende Malonat hydrolysiert.

15. Nitrile, brauchbar bei der Herstellung von Säuren nach Anspruch 1, mit der Formel

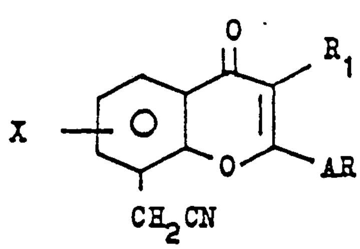

worin AR ein Methyl-, Phenyl-, Methylphenyl-, Methoxyphenyl-, Dimethoxyphenyl-, Benzyl-, Cyclohexyl-, Thenyl-, Furyl- oder Naphtylrest ist und $R_1$ und X Wasserstoff sind.

16. Nitrile, brauchbar bei der Herstellung von Säuren nach Anspruch 2 oder 3, mit der Formel

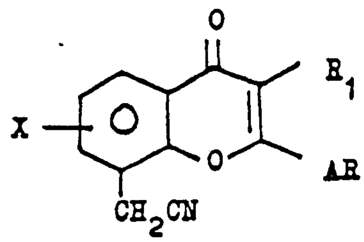

worin AR der Phenylrest ist, $R_1$ Wasserstoff ist und X ein Methyl- oder Methoxyrest ist.

17. Nitril, brauchbar bei der Herstellung einer Verbindung nach Anspruch 5, mit der Formel

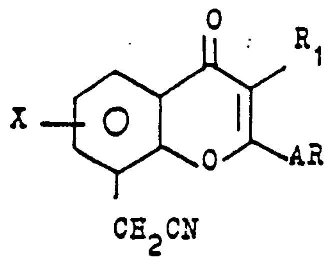

worin AR der Phenylrest ist, $R_1$ der Phenylrest ist und X Wasserstoff ist.

18. Malonat, brauchbar bei der Herstellung von Säuren nach Anspruch 4 mit der Formel

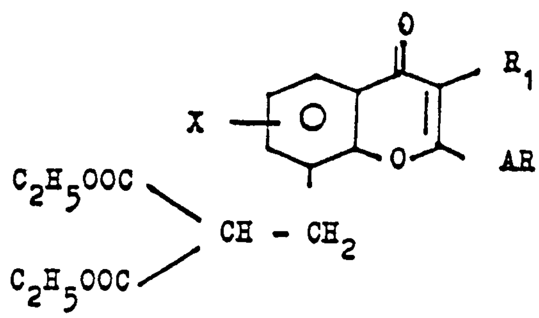

worin AR ein Phenylrest ist, $R_1$ Wasserstoff ist und X Wasserstoff ist.

19. Malonate, brauchbar bei der Herstellung einer Säure nach Anspruch 2 oder 4 mit der Formel

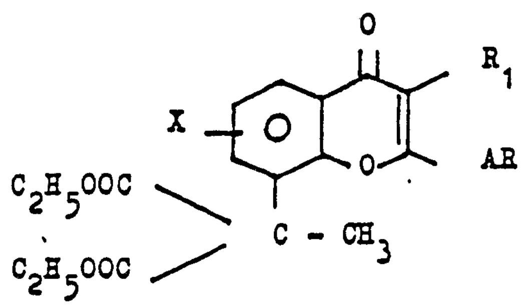

worin AR ein Phenylrest ist, $R_1$ Wasserstoff ist und X der Methylrest ist.

20. (4-Oxo-2-phenyl-4H-[1]-benzopyran-8-yl)-carboxaldehyd und (4-Oxo-3-phenyl-4H-[1]-benzopyran-8-yl)-carboxaldehyd als Zwischenprodukte, die bei der Herstellung von Säuren brauchbar sind.

21. Arzneimittel, enthaltend als Wirkstoff eine Säure oder ein Alkalimetallsalz nach Anspruch 1.

22. Arzneimittel, enthaltend als Wirkstoff eine Säure oder ein Alkalimetallsalz nach Anspruch 2.

23. Arzneimittel, enthaltend als Wirkstoff eine Säure oder ein Alkalimetallsalz nach Anspruch 3.

24. Arzneimittel, enthaltend als Wirkstoff eine Säure oder ein Alkalimetallsalz nach Anspruch 4.

25. Arzneimittel, enthaltend als Wirkstoff eine Säure oder eine Alkalimetallsalz nach Anspruch 5.

26. Arzneimittel, enthaltend als Wirkstoff einen Ester oder Aminoester nach Anspruch 6.

27. Arzneimittel, enthaltend als Wirkstoff einen Ester oder Aminoester nach Anspruch 7.

28. Arzneimittel, enthaltend als Wirkstoff einen Ester oder Aminoester nach Anspruch 8.

29. Arzneimittel, enthaltend als Wirkstoff einen Aminoester nach Anspruch 9.

30. Arzneimittel, enthaltend als Wirkstoff einen Aminoester nach Anspruch 10.

31. Arzneimittel, enthaltend als Wirkstoff einen Aminoester nach Anspruch 11.

32. Arzneimittel, enthaltend als Wirkstoff einen Aminoester nach Anspruch 12.

33. Arzneimittel, enthaltend als Wirkstoff ein Amid nach Anspruch 13.

**Patentansprüche** für den Vertragsstaat AT

1. Verfahren zur Herstellung von 4-Oxo-4H-[1]-benzopyran-8-yl)-alkansäuren der Formel

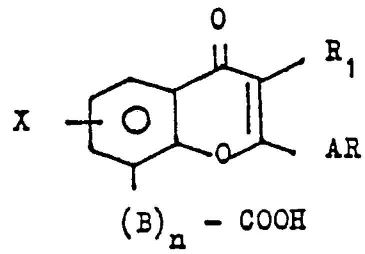

worin AR ein Methyl-, Phenyl-, Methylphenyl-, Methoxyphenyl-, Dimethoxyphenyl-, Thenyl-, Furyl-, Naphthyl-, Cyclohexyl- oder Benzylrest ist, B = $CH_2$, $R_1$ Wasserstoff ist, X Wasserstoff ist und n = 1, dadurch gekennzeichnet, daß man die entsprechenden Nitrile hydrolysiert.

2. Verfahren zur Herstellung von (4-Oxo-4H-[1]-benzopyran-8-yl)-alkansäuren der Formel

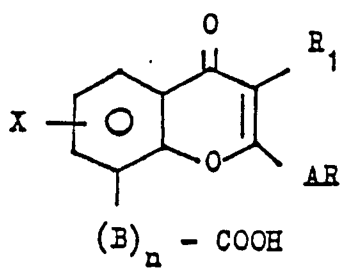

worin AR ein Phenylrest, ist $R_1$ Wasserstoff ist, X ein Methylrest ist, n = 1, B = $CH_2$, $CH_2$-$CH_2$ oder

$CH_3-CH$,

dadurch gekennzeichnet, daß man, wenn B = $CH_2$, das entsprechende Nitril hydrolysiert, wenn B =

$CH_3-CH$,

die Einführung des verzweigten Restes durch Überführung der Säure in einen Ester und dann in ein Malonat, das alkyliert und dann zu der entsprechenden Säure hydrolysiert wird, realisiert, und wenn B = $CH_2$-$CH_2$, ein 8-Brommethylbenzo-pyranon-4 mit Diethylmalonat in Gegenwart eines basischen Mittels reagieren läßt, worauf das erhaltene substituiert wird.

3. Verfahren zur Herstellung von (4-Oxo-4H-[1]-benzopyran-8-yl)-alkansäuren der Formel

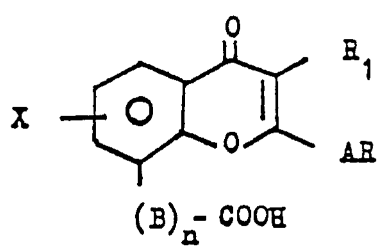

worin AR ein Phenylrest ist, $R_1$ Wasserstoff ist, B = $CH_2$, n = 1, X = der Hydroxy- oder Methoxyrest, dadurch gekennzeichnet, daß man die entsprechenden Nitrile hydrolysiert.

4. Verfahren zur Herstellung von (4-Oxo-4H-[1]-benzopyran-8-yl)-alkansäuren der Formel

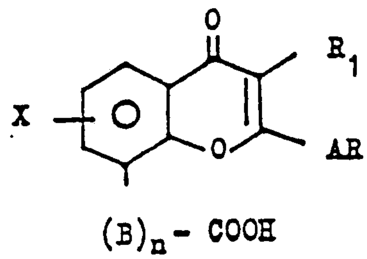

worin AR ein Phenylrest ist, B =

$$CH_3-CH$$

oder CH=CH, $R_1$ und X Wasserstoff sind und n = 1, <u>dadurch gekennzeichnet</u>, daß man, wenn B =

$$CH_3-CH,$$

die Einführung des verzweigten Restes durch Umwandlung der Säure in einen Ester und dann in ein Malonat, das alkyliert und dann zur erwünschten Säure hydrolysiert wird, realisiert und, wenn B = CH=CH, ein 8-Brommethylbenzopyranon-4 mit Hexamethylentetramin zur Umsetzung bringt und dann den erhaltenen Aldehyd der Kondensation mit Essigsäureanhydrid in Gegenwart von Natriumacetat unterzieht.

5. Verfahren zur Herstellung von (4-Oxo-4H-[1]-benzopyran-8-yl)-alkansäuren der Formel

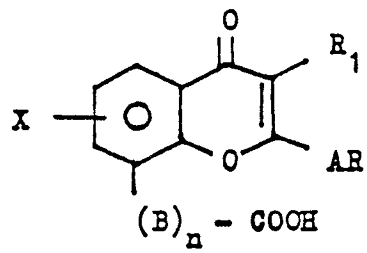

worin AR ein Phenylrest oder Wasserstoff ist, n = 1, $R_1$ = ein Phenylrest, x Wasserstoff ist und B = $CH_2$, <u>dadurch gekennzeichnet</u>, daß man die entsprechenden Nitrile hydrolysiert.

6. Verfahren zur Herstellung von Nitrilen, die bei der Herstellung von Säuren nach Anspruch 1 brauchbar sind, mit der Formel

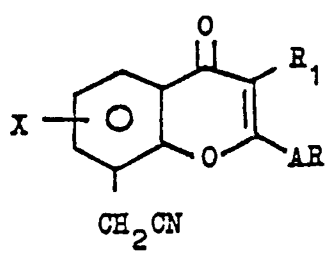

worin AR ein Methyl-, Phenyl-, Methylphenyl-, Methoxyphenyl-, Dimethoxyphenyl-, Benzyl-, Cyclohexyl-, Thenyl-, Furyl- oder Naphthylrest ist, $R_1$ und X Wasserstoff sind, <u>dadurch gekennzeichnet</u>, daß man ein 8-Brommethylbenzopyranon-4 der Formel

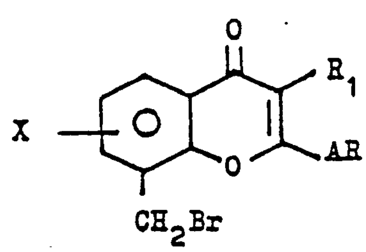

worin AR, $R_1$ und X die gleichen Bedeutungen wie oben haben, mit einem Alkalicyanid behandelt.

7. Verfahren zur Herstellung von Nitrilen, die bei der Herstellung von Säuren nach Anspruch 2 oder 3 brauchbar sind, mit der Formel

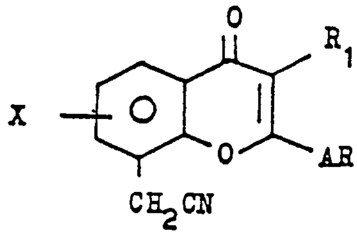

worin AR der Phenylrest ist, $R_1$ Wasserstoff ist, X ein Methyl- oder Methoxyrest ist, dadurch gekennzeichnet, daß man ein 8-Brommethylbenzopyranon-4 der Formel

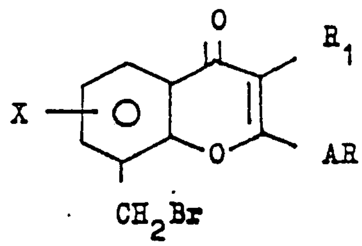

worin AR, $R_1$ und X die gleichen Bedeutungen wie oben haben, mit einem Alkalicyanid behandelt.

8. Verfahren zur Herstellung von Nitrilen, die bei der Herstellung einer Verbindung nach Anspruch 5 brauchbar sind, mit der Formel

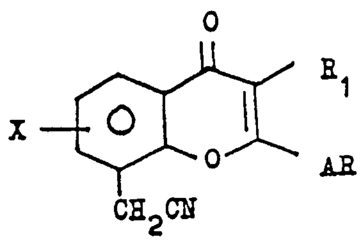

worin AR der Phenylrest ist, $R_1$ der Phenylrest ist und X Wasserstoff ist, dadurch gekennzeichnet, daß man das 8-Brommethylbenzopyranon-4 der Formel

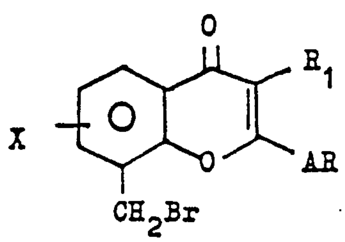

worin AR, $R_1$ und X die gleichen Bedeutungen wie oben haben, mit einem Alkalicyanid behandelt.

9. Verfahren zur Herstellung eines Malonats, das bei der Herstellung von Säuren nach Anspruch 4 brauchbar ist, mit der Formel

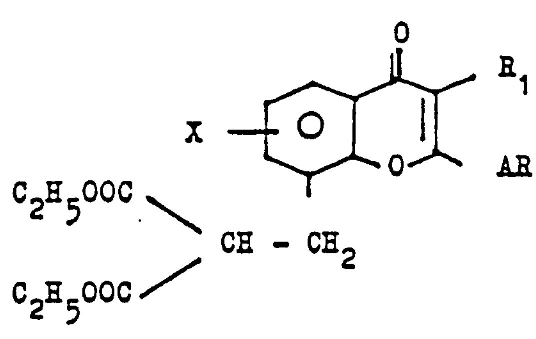

worin AR ein Phenylrest ist, $R_1$ Wasserstoff ist und X Wasserstoff repräsentiert, gekennzeichnet durch die Umwandlung einer Säure in einen Ester und dann in ein Malonat.

10. Verfahren zur Herstellung von Malonaten, die bei der Herstellung einer Säure nach Anspruch 2 oder 4

brauchbar sind, mit der Formel

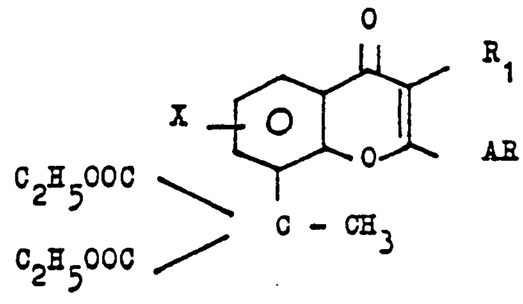

worin AR ein Phenylrest ist, $R_1$ Wasserstoff ist und X der Methylrest ist, gekennzeichnet durch die Umwandlung einer Säure in einen Ester und dann in ein Malonat.

11. Verfahren zur Herstellung von (4-Oxo-2-phenyl-4H-[1]-benzopyran-8-yl)-carboxaldehyd und (4-Oxo-3-phenyl-4H-[1]-benzopyran-8-yl)-carboxaldehyd als Zwischenprodukte, die bei der Herstellung von Säuren brauchbar sind, gekennzeichnet durch die Kondensation eines 8-Brommethylbenzopyranon-4 mit Hexamethylentetramin.

12. Verfahren zur Herstellung von (2-N,N'-Diethylamino-ethyl)-(4-oxo-2-phenyl-4H-[1]-benzopyran-8-yl)-acetamid, gekennzeichnet durch die Kondensation von Ethyl-(4-oxo-2-phenyl-4H-[1]-benzopyran-8-yl)-acetat mit 2-Diethyl-aminoethylamin.

13. Verfahren zur Herstellung von Alkalimetallsalzen von Säuren nach den Ansprüchen 1 bis 5, gekennzeichnet durch die Neutralisation der obigen Säuren.

14. Verfahren zur Herstellung von Estern und Aminoestern einer Verbindung nach Anspruch 1 der Formel

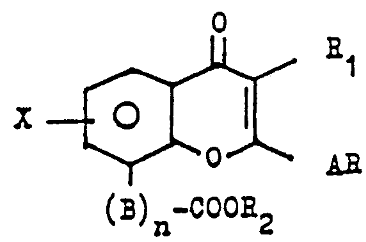

worin AR ein Phenylrest ist, $R_1$ Wasserstoff ist, X Wasserstoff ist, n = 1, B = $CH_2$, $R_2$ ein Methyl-, Ethyl-, Hydroxyethyl-, Diethylaminoethyl-, Dimethylaminopropyl- oder Morpholinoethylrest ist, dadurch gekennzeichnet, daß die Ester durch Veresterung der obigen Säuren mit einem Alkohol und die Aminoester durch Kondensation dieser Säuren mit einem Chloralkylaminodialkyl gemäß den Bezeichnungen des Substituenten $R_2$ erhalten werden.

15. Verfahren zur Herstellung von Estern und Aminoestern einer Verbindung nach Anspruch 1 der Formel

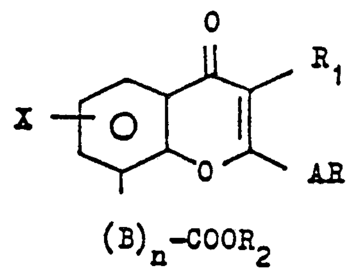

worin AR ein Thenylrest ist, $R_1$ Wasserstoff ist, X Wasserstoff ist, n = 1, B = $CH_2$ und $R_2$ ein Methyl- oder Dimethylaminoethylrest ist, gekennzeichnet durch Veresterung der Säure mit Methanol und im Fall des Aminoesters durch Kondensation der Säure mit Chlorethylaminodiethyl.

16. Verfahren zur Herstellung von Estern und Aminoestern einer Verbindung nach Anspruch 2 der Formel

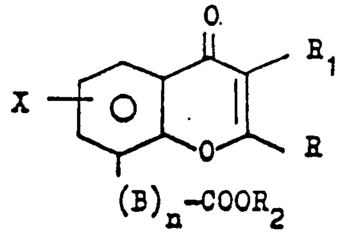

worin AR ein Phenylrest ist, $R_1$ Wasserstoff ist, n = 1, B = $CH_2$, X ein Methylrest ist und $R_2$ ein Methyl- oder Diethylaminoethylrest ist, gekennzeichnet durch Veresterung der Säure mit dem Methanol und im Falle des Aminoesters durch Kondensation der Säure mit Chlorethylaminodiethyl.

17. Verfahren zur Herstellung eines Aminoesters einer Verbindung nach Anspruch 2 der Formel

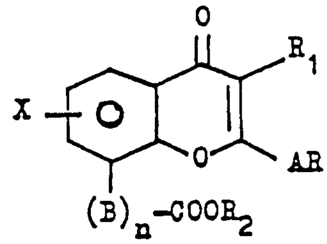

worin AR ein Phenylrest ist, $R_1$ Wasserstoff ist, B =

FIG100/15

n = 1, X Wasserstoff ist und $R_2$ der Diethylaminoethylrest ist, gekennzeichnet durch die Kondensation der Säure mit Chlorethylaminodiethyl.

18. Verfahren zur Herstellung von Aminoestern einer Verbindung nach Anspruch 4 der Formel

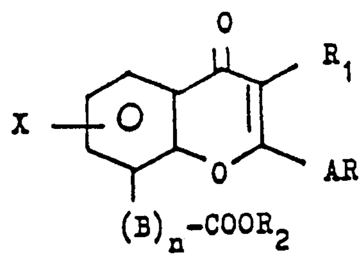

worin AR ein Phenylrest ist, $R_1$ und X Wasserstoff sind, B = CH=CH, n = 1 und $R_2$ ein Diethylaminoethyl- oder Morpholinoethylrest ist, dadurch gekennzeichnet, daß die Säure mit Chlorethylaminodiethyl oder Chlorethylmorpholin kondensiert wird.

19. Verfahren zur Herstellung einer Verbindung der Formel

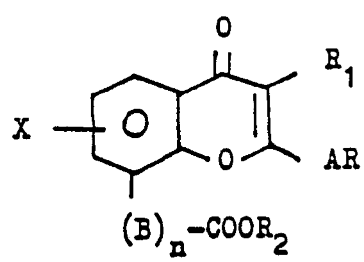

worin AR ein Phenylrest ist, $R_1$ = Wasserstoff, X ein Methoxyrest ist, B = $CH_2$ n = 1 und $R_2$ der Diethylaminoethylrest ist, dadurch gekennzeichnet, daß die Säure mit Chlorethylaminodiethyl kondensiert wird.

20. Verfahren zur Herstellung von Verbindungen der Formel

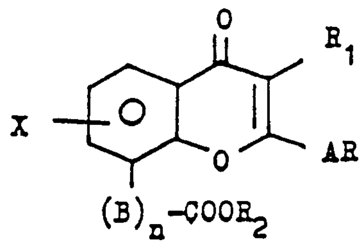

worin AR ein Methylphenyl- oder Methoxyphenylrest ist, $R_1$ und X Wasserstoff sind, $B = CH_2$, $n = 1$ und $R_2$ der Diethylaminoethylrest ist, dadurch gekennzeichnet, daß die Säure mit Chlorethylaminodiethyl kondensiert wird.

**Claims** for the contracting States: BE, CH, DE, FR, GB, IT, LI, LU, NL, SE

1. [4-Oxo-4H-[1]-benzopyran-8-yl]-alkanoic acids, characterised by the formula

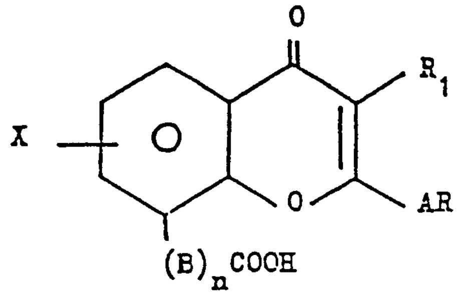

in which AR is a methyl, phenyl, methylphenyl, methoxyphenyl, dimethoxyphenyl, thenyl, furyl, naphthyl, cyclohexyl or benzyl radical, $B = CH_2$; $R_1$ is hydrogen, X is hydrogen, $n = 1$, as well as their alkali metal salts.

2. [4-Oxo-4H-[1]-benzopyran-8-yl]-alkanoic acids, characterised by the formula

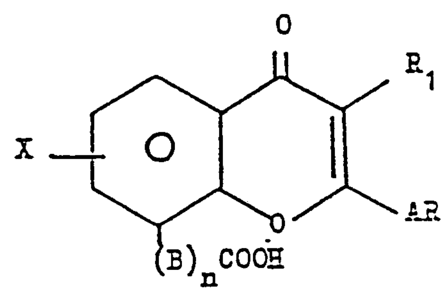

in which AR is a phenyl radical, $R_1$ is hydrogen, X is a methyl radical, $n = 1$, $B = CH_2$ or $CH_3$-$_1CH$, as well as their alkali metal salts.

3. (4-Oxo-4H-[1]-benzopyran-8-yl]-alkanoic acids, characterised by the formula

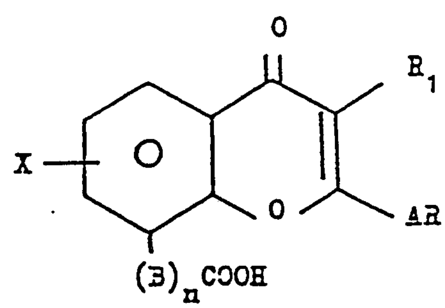

in which AR is a phenyl radical, $R_1$ is hydrogen, $B = CH_2$, $n = 1$, X = the hydroxy or methoxy radical, as well as their alkali metal salts.

4. (4-Oxo-4H-[1]-benzopyran-8-yl]-alkanoic acids, characterised by the formula

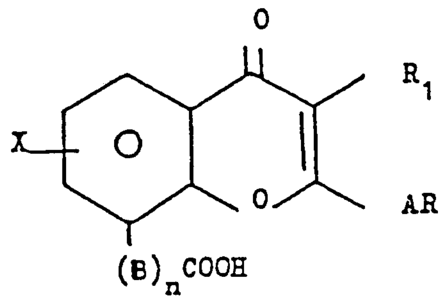

in which AR is a phenyl radical, B =

$CH_3-CH$

or $CH_2$-$CH_2$ or CH = CH; $R_1$ and X are hydrogen, n = 1, as well as their alkali metal salts.

5. (4-Oxo-4H-[1]-benzopyran-8-yl] alkanoic acids, characterised by the formula

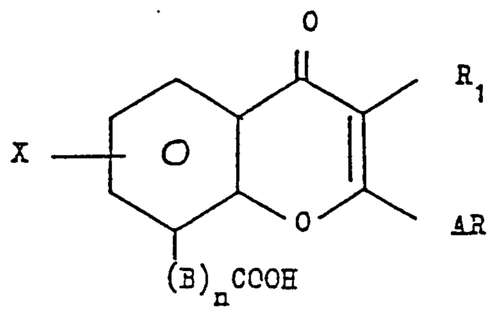

in which AR is a phenyl radical or hydrogen, n = 1, $R_1$ = a phenyl radical, X is hydrogen, B = $CH_2$, as well as their alkali metal salts.

6. Esters and aminoesters of a compound according to claim 1, characterised by the formula

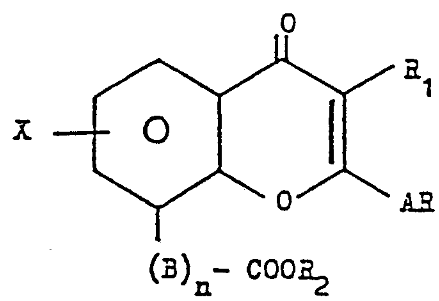

in which AR is a phenyl radical, $R_1$ = hydrogen, X is hydrogen, n = 1, B = $CH_2$, $R_2$ is a methyl, ethyl, hydroxyethyl, diethylaminoethyl, dimethylaminopropyl or morpholinoethyl radical.

7. Esters and aminoesters of a compound according to claim 1 of the formula

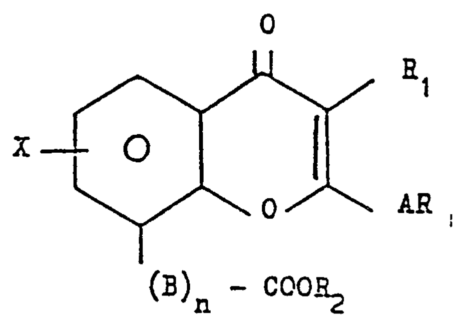

in which AR is a thenyl radical, $R_1$ = hydrogen, X is hydrogen, n = 1, B = $CH_2$, $R_2$ is a methyl or diethylaminoethyl radical.

8. Esters and aminoesters of a compound according to claim 2 of the formula

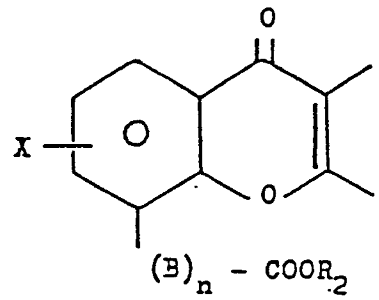

in which AR is a phenyl radical, $R_1$ is hydrogen, n = 1, B = $CH_2$, X = methyl radical and $R_2$ is a methyl or diethylaminoethyl radical.

9. Aminoester of a compound according to claim 2 of the formula

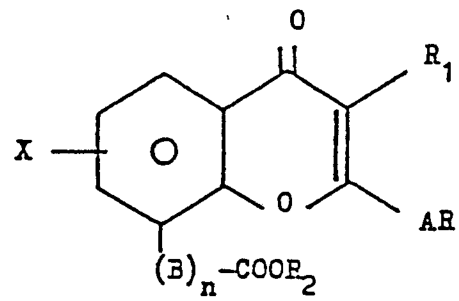

in which AR is a phenyl radical, $R_1$ is hydrogen, B =

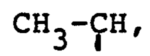

n = 1, X = hydrogen and $R_2$ is the diethylaminoethyl radical.

10. Aminoesters of a compound according to claim 4 of the formula

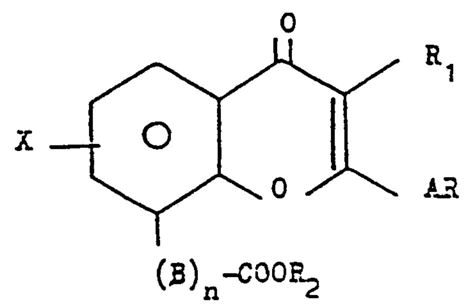

in which AR is a phenyl radical, $R_1$ and X are hydrogen, B = CH = CH, n = 1, $R_2$ is a diethylaminoethyl or morpholinoethyl radical.

11. Aminoester of a compound according to claim 3 of the formula

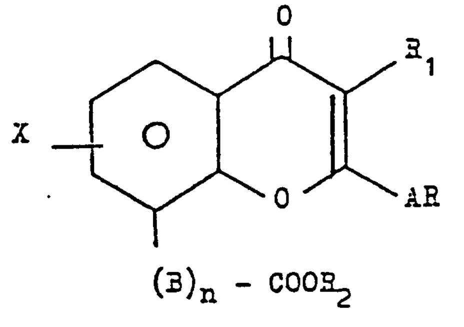

in which AR is a phenyl radical, $R_1$ = hydrogen, X a methoxy radical B = $CH_2$, n = 1 and $R_2$ is the diethylaminoethyl radical.

12. Aminoesters of a compound according to claim 1 of the formula

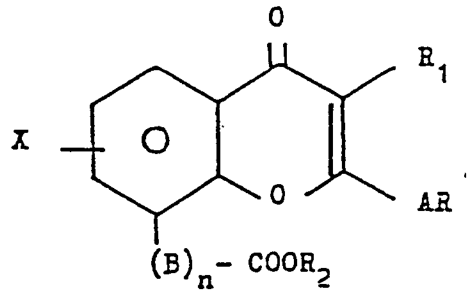

in which AR is a methylphenyl or methoxyphenyl radical, $R_1$ and X = hydrogen, B = $CH_2$, n = 1 and $R_2$ is the diethylaminoethyl radical.

13. Amide of a compound according to claim 1 of the formula

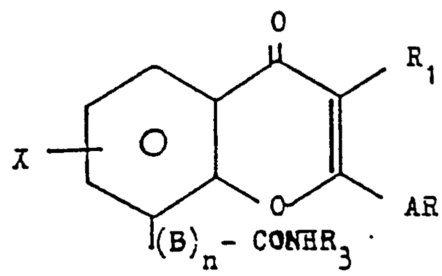

in which AR is a phenyl radical, $R_1$ and X are hydrogen, B = $CH_2$, n = 1, $R_3$ is the diethylaminoethyl radical.

14. Process for preparing acids according to any one of the claims 1 to 5, characterised in that when B = $CH_2$ the corresponding nitriles are hydrolyzed, the said nitriles being obtained by treating the corresponding bromo-methyl-8-benzopyranone-4 with an alkaline cyanide; when B : CH = CH, the corresponding bromomethyl-8-benzopyranone-4 is made to react with hexamethylenetetramine, followed by condensation of the aldehyde obtained with acetic anhydride in the presence of sodium acetate; when B is a radical $CH_2$-$CH_2$ or

FIG119/15

the corresponding malonate is hydrolyzed.

15. Nitriles which may be used in the preparation of the acids according to claim 1, represented by the formula

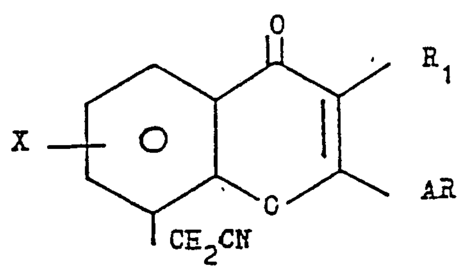

in which AR is a methyl, phenyl, methylphenyl, methoxyphenyl, dimethoxyphenyl, benzyl, cyclohexyl, thenyl, furyl, naphthyl radical, $R_1$ and X are hydrogen.

16. Nitriles which may be used in the preparation of the acids according to claim 2 or 3 represented by the formula

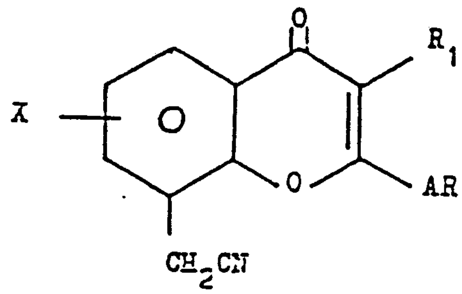

in which AR is the phenyl radical, $R_1$ is hydrogen, X is a methyl or methoxy radical.

17. Nitrile which may be used in the preparation of a compound according to claim 5, represented by the formula

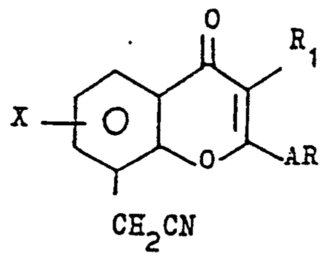

in which AR is the phenyl radical, $R_1$ is the phenyl radical and X is hydrogen.

18. Malonate which may be used in the preparation of the acids according to claim 4, represented by the formula

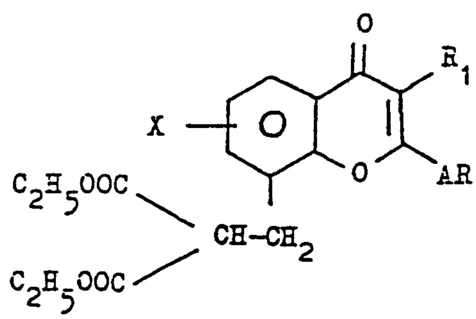

in which AR is a phenyl radical, $R_1$ hydrogen, X represents hydrogen.

19. Malonates which may be used in the preparation of an acid according to claim 2 or 4, represented by the formula

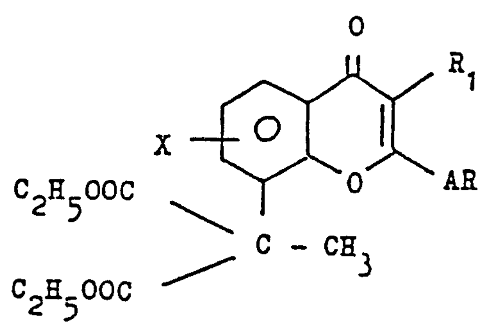

in which AR is a phenyl radical, $R_1$ hydrogen, X the methyl radical.

20. [4-Oxo-2-phenyl-4H-[1]-benzopyran-8-yl] carboxaldehyde and [4-oxo-3-phenyl-4H-[1]-benzopyran-8-yl] carboxaldehyde as intermediate products which may be used in the preparation of acids.

21. Medicine containing as an active principle an acid or an alkali metal salt according to claim 1.

22. Medicine containing as an active principle an acid or an alkali metal salt according to claim 2.

23. Medicine containing as an active principle an acid or an alkali metal salt according to claim 3.

24. Medicine containing as an active principle an acid or an alkali metal salt according to claim 4.

25. Medicine containing as an active principle an acid or an alkali metal salt according to claim 5.

26. Medicine containing as an active principle an ester or aminoester according to claim 6.

27. Medicine containing as an active principle an ester or aminoester according to claim 7.

28. Medicine containing as an active principle an ester or aminoester according to claim 8.
29. Medicine containing as an active principle an aminoester according to claim 9.
30. Medicine containing as an active principle an aminoester according to claim 10.
31. Medicine containing as an active principle an aminoester according to claim 11.
32. Medicine containing as an active principle an aminoester according to claim 12.
33. Medicine containing as an active principle an amide according to claim 13.

**Claims** for the contracting State: AT

1. Process for preparing (4-oxo-4H-[1]-benzopyran-8-yl]-alkanoic acids or the formula

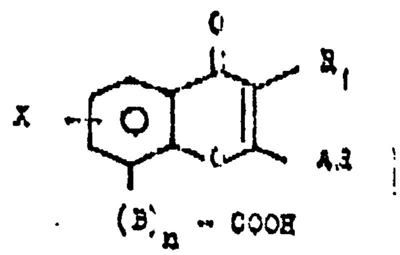

in which AR is a methyl, phenyl, methylphenyl, methoxyphenyl, dimethoxyphenyl, thenyl, furyl, naphthyl, cyclohexyl or benzyl radical, B = $CH_2$; $R_1$ is hydrogen, X is hydrogen, n = 1, characterised in that the corresponding nitriles are hydrolyzed.

2. Process for preparing (4-oxo-4H-[1]-benzopyran-8-yl]-alkanoic acids of the formula

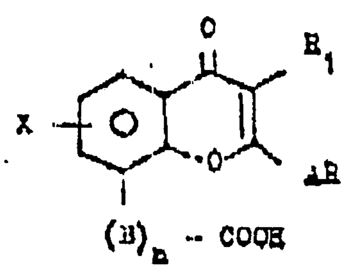

in which AR is a phenyl-radical, $R_1$ is hydrogen, X is a methyl radical, n = 1, B = $CH_2$, $CH_2$-$CH_2$ or

$$CH_3-CH,$$

characterised in that when B = $CH_2$ the corresponding nitrile is hydrolyzed; when B =

$$CH_3-CH$$

the introduction of the branched radical is performed by converting the acid into an ester and then into a malonate which is alkylated and then hydrolyzed in corresponding acid; when B = $CH_2$-$CH_2$ bromo-methyl-8-benzopyranone-4 is made to react with diethyl malonate in the presence of a basic agent and then the substituted malonate obtained is hydrolyzed in corresponding acid.

3. Process for preparing [4-oxo-4H-[1]-benzopyran-8-yl]-alkanoic acids of the formula

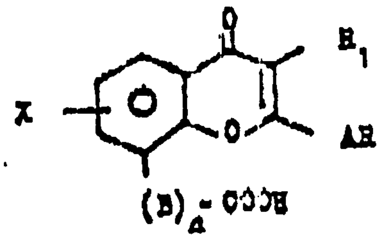

in which AR is a phenyl radical, $R_1$ is hydrogen, B = $CH_2$, n = 1, X = the hydroxy or methoxy radical, characterised in that the corresponding nitriles are hydrolyzed.

4. Process for preparing [4-oxo-4H-[1]-benzopyran-8-yl]-alkanoic acids of the formula

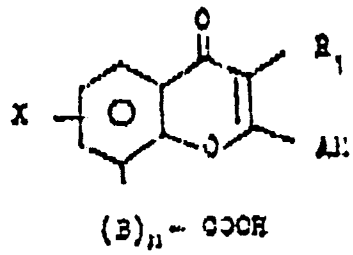

in which AR is a phenyl radical, B =

$CH_3-CH$

or CH = CH; $R_1$ and X are hydrogen, n = 1, characterised in that when B =

$CH_3-CH$

the introduction of the branched radical is performed by converting the acid into an ester and then into a malonate which is alkylated and then hydrolyzed in the expected acid and when B = CH = CH bromo-methyl-8-benzopyranone-4 is made to react with hexamethylene-tetramine followed by condensation of the aldehyde obtained with acetic anhydride in the presence of sodium acetate.

5. Process for preparing [4-oxo-4H-[1]-benzopyran-8-yl] alkanoic acids of the formula

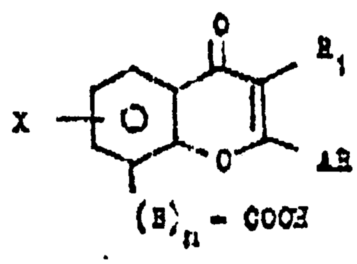

in which AR in a phenyl radical or hydrogen, n = 1, $R_1$ = a phenyl radical, X is hydrogen, B = $CH_2$, characterised in that the corresponding nitriles are hydrolyzed.

6. Process for preparing nitriles which may be used in the preparation of the acids according to claim 1, represented by the formula

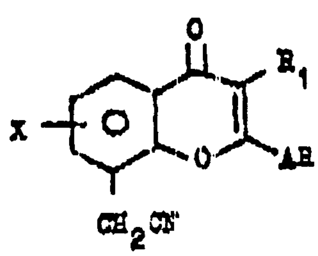

in which AR is a methyl, phenyl, methylphenyl, methoxyphenyl, dimethoxyphenyl, benzyl, cyclohexyl, thenyl, furyl, naphthyl radical, $R_1$ and X are hydrogen, characterised in that a bromo-methyl-8-benzopyranone-4 of the formula

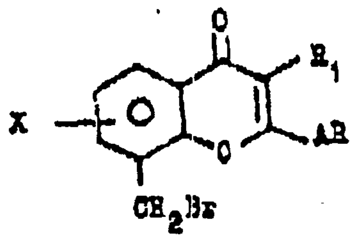

in which AR, $R_1$ and X have the same meanings as above, is treated with an alkaline cyanide.

7. Process for preparing nitriles which may be used in the preparation of the acids according to claim 2 or 3 represented by the formula

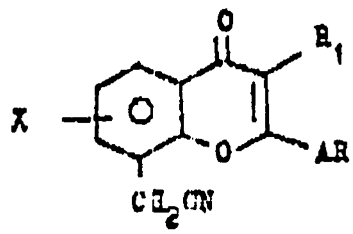

in which AR is the phenyl radical, $R_1$ is hydrogen, X is a methyl or methoxy radical, characterised in that a bromo-methyl-8-benzopyranone-4 of the formula

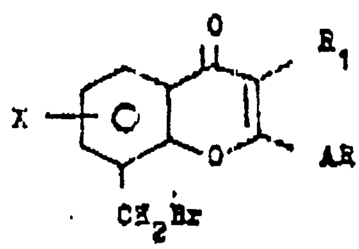

in which AR, $R_1$ and X have the same meanings as above, is treated with an alkaline cyanide.

8. Process for preparing nitriles which may be used in the preparation of a compound according to claim 5, represented by the formula

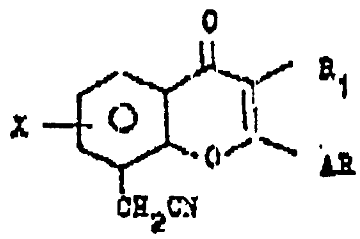

in which AR is the phenyl radical, $R_1$ is the phenyl radical and X is hydrogen, characterised in that the bromo-methyl-8-benzopyranone-4 of the formula

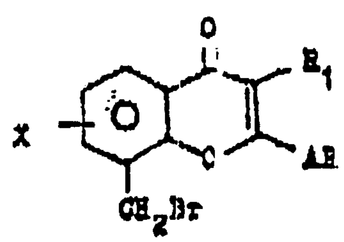

in which AR, $R_1$ and X have the same meanings as above, is treated with an alkaline cyanide.

9. Process for preparing a malonate which may be used in the preparation of the acids according to claim 4, represented by the formula

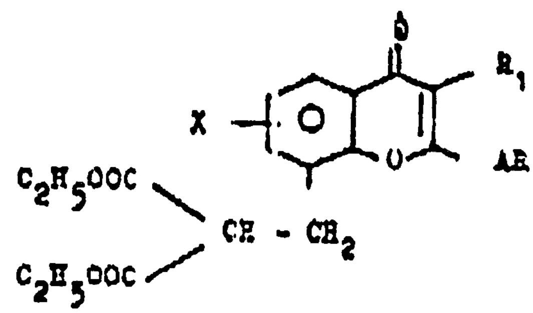

in which AR is a phenyl radical, $R_1$ is hydrogen, X represents hydrogen, characterised by the conversion of an acid into an ester and then into a malonate.

10. Process for preparing malonates which may be used in the preparation of an acid according to claim 2 or 4, represented by the formula.

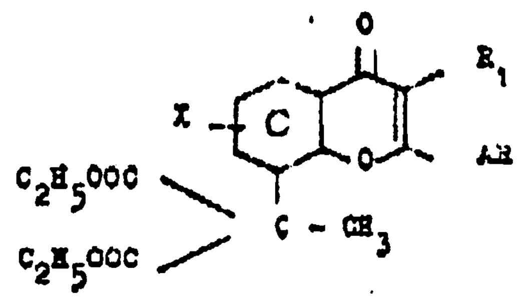

in which AR is a phenyl radical, $R_1$ is hydrogen, X is the methyl radical, characterised by the conversion of an acid into an ester and then into a malonate.

11. Process for preparing [4-oxo-2-phenyl-4H-[1]-benzopyran-8-yl] carboxaldehyde and [4-oxo-3-phenyl-4H-[1]-benzopyran-8-yl] carboxaldehyde as intermediate products which may be used in the preparation of the acids, characterised by the condensation or a bromo-methyl-8-benzopyranone-4 with hexamethylene-tetramine.

12. Process for preparing N-[N,N'-diethylamino-2-ethyl] [4-oxo-2-phenyl-4H-[1]-benzopyran-8-yl] acetamide, characterised by the condensation of the [4-oxo-2-phenyl-4H-[1]-benzopyran-8-yl] ethyl acetate with diethylamino-2-ethylamine.

13. Process for preparing the alkali metal salts of the acids according to claim 1 to 5, characterised by the neutralization of the previous acids.

14. Process for preparing esters and aminoesters of a compound according to claim 1 of the formula

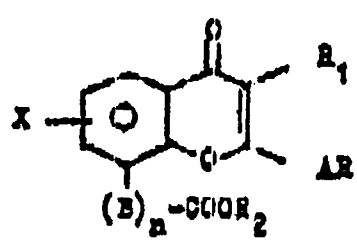

in which AR is a phenyl radical, $R_1$ is hydrogen, X is hydrogen, n = 1, B = $CH_2$, $R_2$ is a methyl, ethyl, hydroxyethyl, diethylaminoethyl, dimethylaminopropyl or morpholinoethyl radical, characterised in that the esters are obtained through esterification by an alcohol of the previous acids and the aminoesters are obtained through condensation of said acids with a chloroalkyl aminodialkyl according to the meanings of the substituent $R_2$.

15. Process for preparing esters and aminoesters of a compound according to claim 1 of the formula

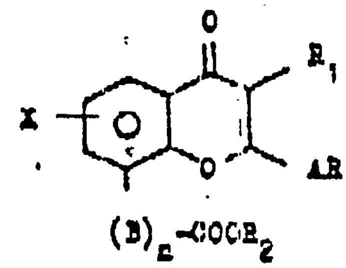

in which AR is a thenyl radical, $R_1$ is hydrogen, X is hydrogen, n = 1, B = $CH_2$, $R_2$ is a methyl or diethylaminoethyl radical characterised by the esterification of the acid by methanol and in the case of the aminoester by condensation of the acid with chloroethyl aminodiethyl.

16. Process for preparing esters and aminoesters of a compound according to claim 2 of the formula

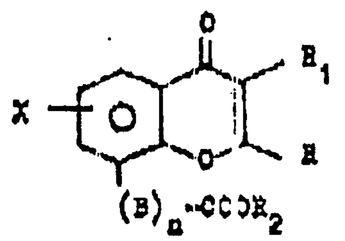

FIG144/35

in which AR is a phenyl radical, $R_1$ is hydrogen, n = 1, B = $CH_2$, X is a methyl radical and $R_2$ is a methyl or diethylaminoethyl radical, characterised by the esterification of the acid by methanol and in the case of the aminoester by the condensation of the acid with chloroethyl aminodiethyl.

17. Process for preparing an aminoester of a compound according to claim 2 of the formula

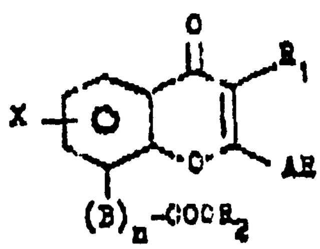

in which AR is a phenyl radical, $R_1$ is hydrogen, B =

$$CH_3-\underset{|}{C}H,$$

n = 1, X is hydrogen and $R_2$ is the diethylaminoethyl radical characterised by the condensation of the acid with chloroethyl aminodiethyl.

18. Process for preparing aminoesters or a compound according to claim 4 of the formula

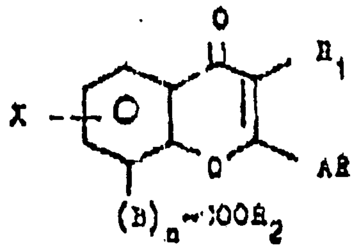

in which AR is a phenyl radical, $R_1$ and X are hydrogen, B = CH = CH, n = 1, $R_2$ is a diethylaminoethyl or morpholinoethyl radical, characterised in that the acid is condensed with chloroethyl aminodiethyl or chloroethyl morpholine.

19. Process for preparing a compound of the formula

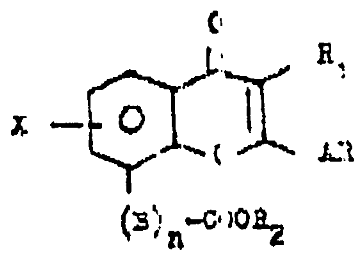

in which AR is a phenyl radical, $R_1$ = hydrogen, X a methoxy radical, B = $CH_2$, n = 1 and $R_2$ is the diethylaminoethyl radical, characterised in that the acid is condensed with chloroethyl aminodiethyl.

20. Process for preparing compounds of the formula

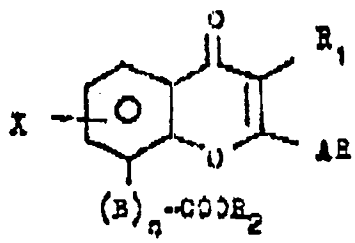

in which AR is a methylphenyl or methoxyphenyl radical, $R_1$ and X are hydrogen, B = $CH_2$, n = 1 and $R_2$ is the diethylaminoethyl radical, characterised in that the acid is condensed with chloroethyl aminodiethyl.

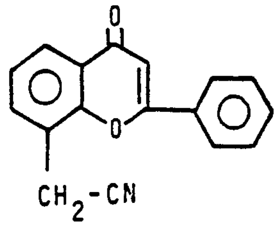

FORMULE 1

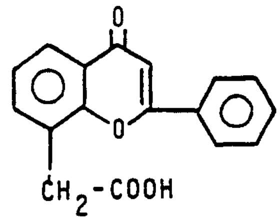

FORMULE 2

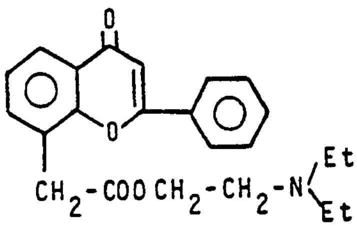

FORMULE 3

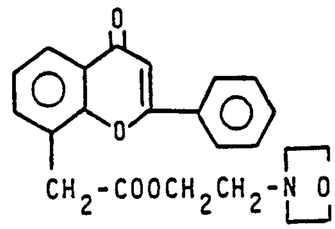

FORMULE 4

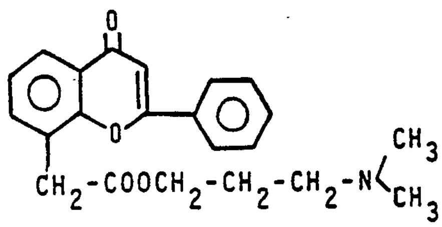

FORMULE 5

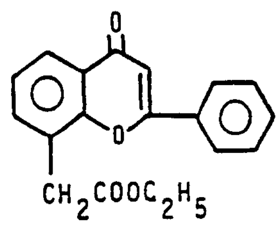

FORMULE 6

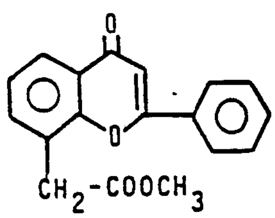

FORMULE 7

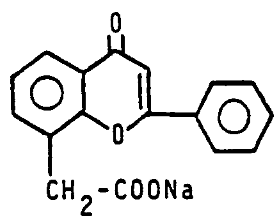

FORMULE 8

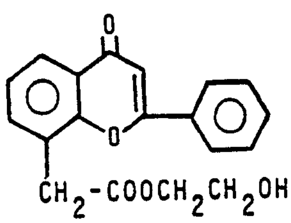

FORMULE 9

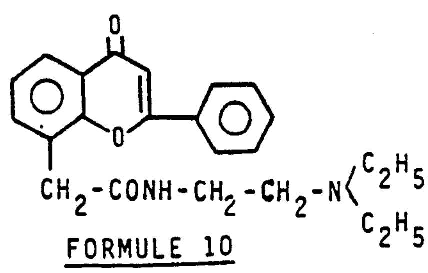

FORMULE 10

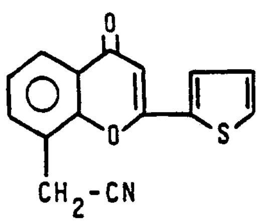

FORMULE 11

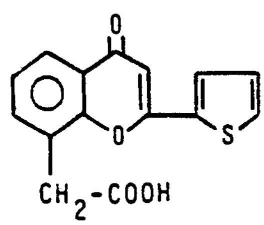

FORMULE 12

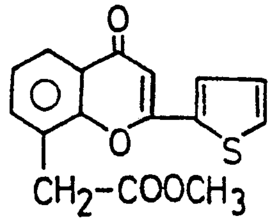

FORMULE 13

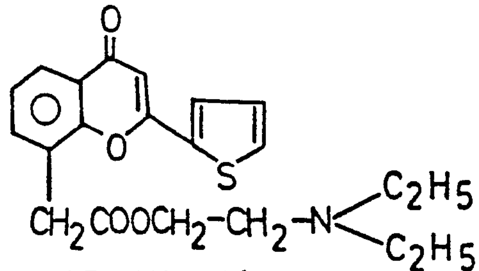

FORMULE 14

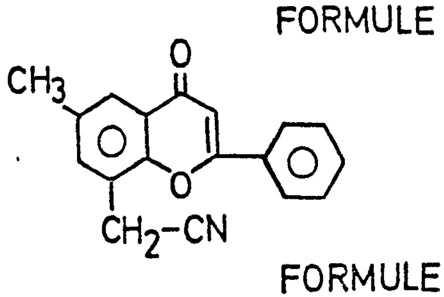

FORMULE 15

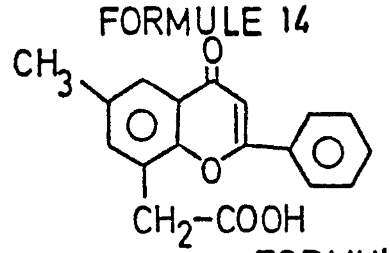

FORMULE 16

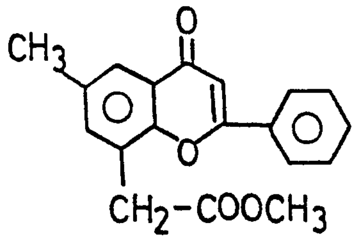

FORMULE 17

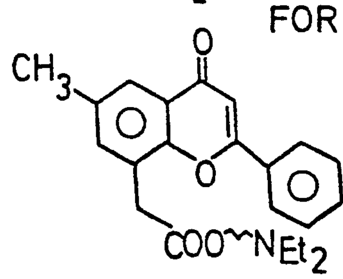

FORMULE 18

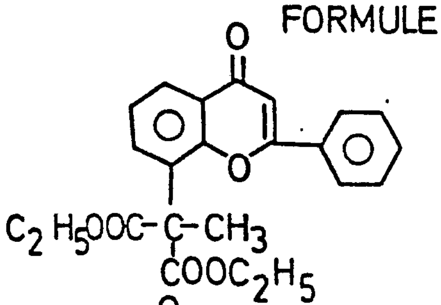

FORMULE 19

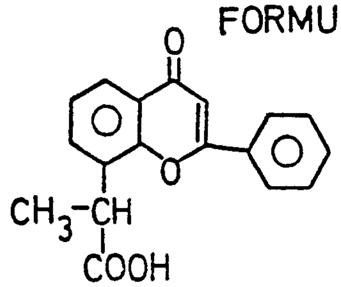

FORMULE 20

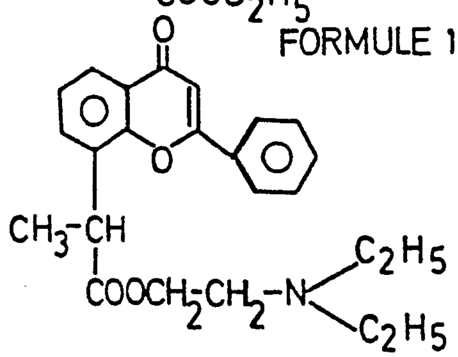

FORMULE 21

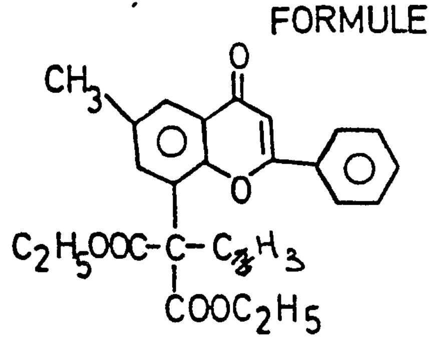

FORMULE 22

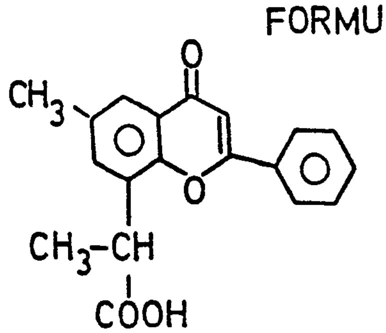

FORMULE 23

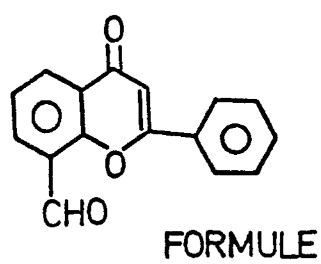

FORMULE 24

3/5

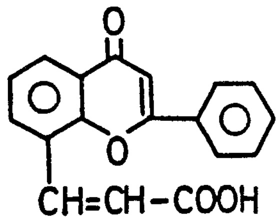

FORMULE 25

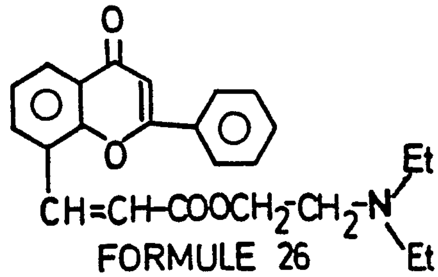

FORMULE 26

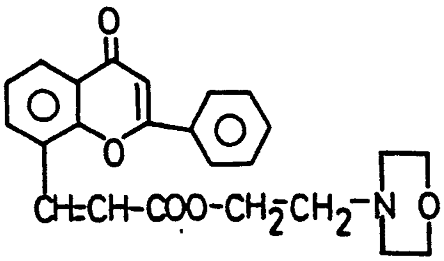

FORMULE 27

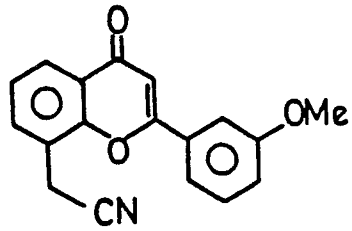

FORMULE 28

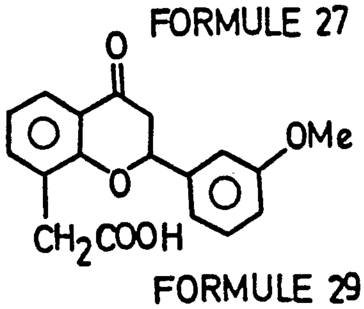

FORMULE 29

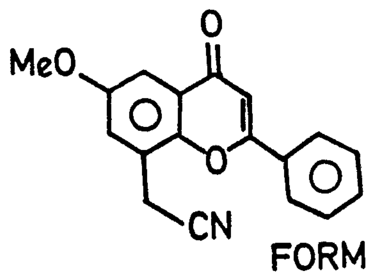

FORMULE 30

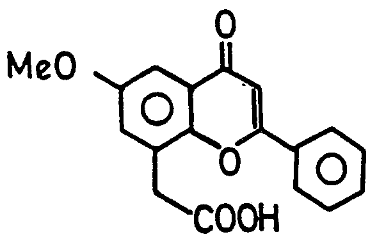

FORMULE 31

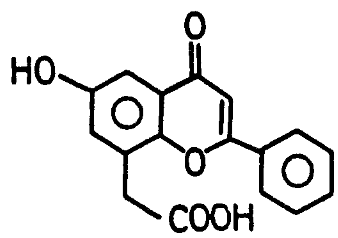

FORMULE 32

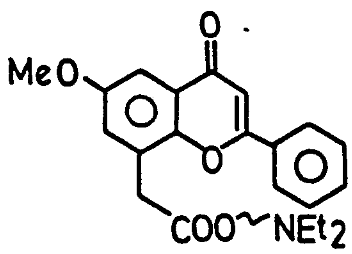

FORMULE 33

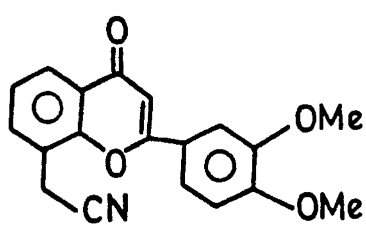

FORMULE 34

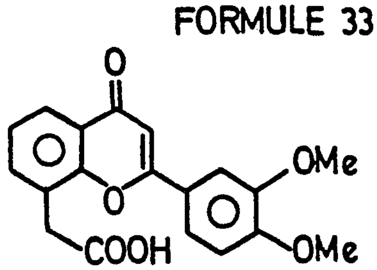

FORMULE 35

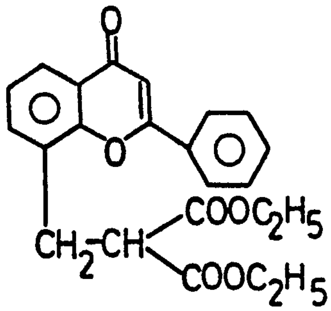

FORMULE 36

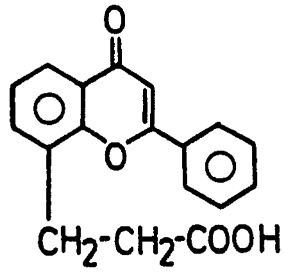

FORMULE 37

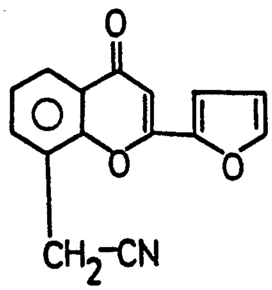

FORMULE 38

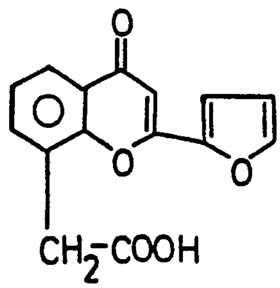

FORMULE 39

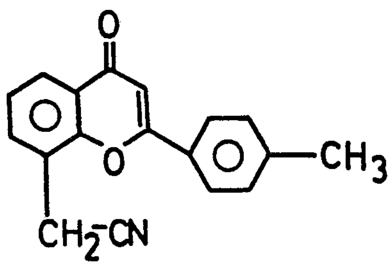

FORMULE 40

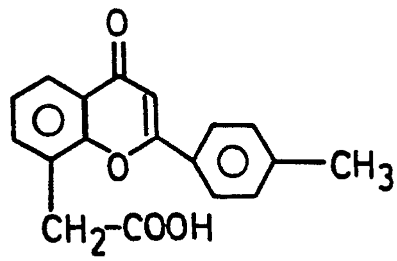

FORMULE 41

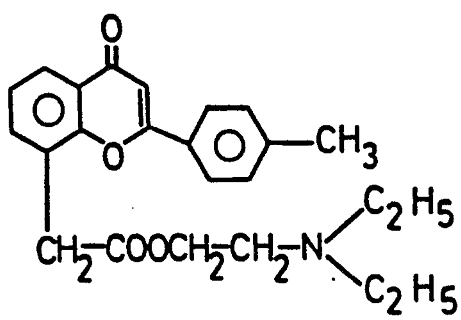

FORMULE 42

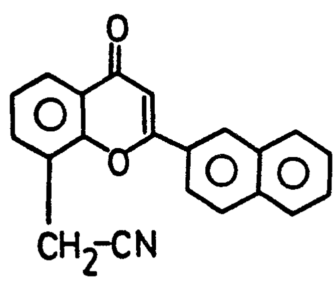

FORMULE 43

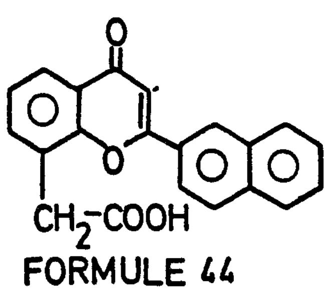

FORMULE 44

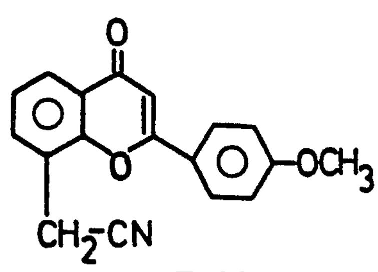

FORMULE 45

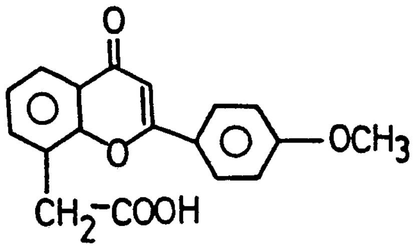

FORMULE 46

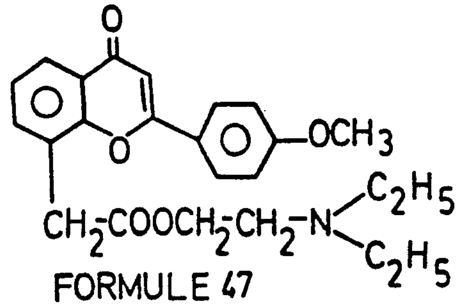

FORMULE 47

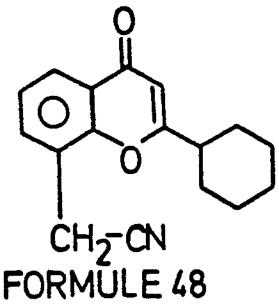

FORMULE 48

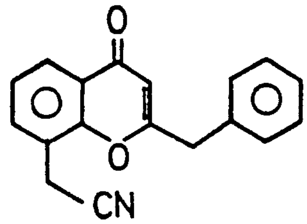

FORMULE 49

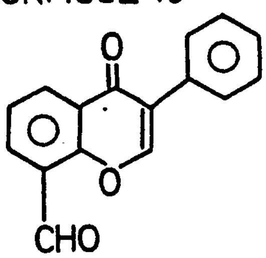

FORMULE 50

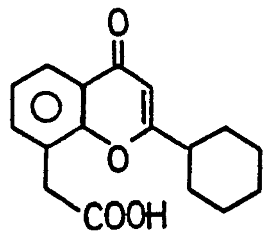

FORMULE 51

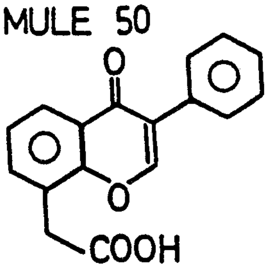

FORMULE 52

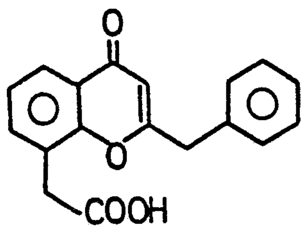

FORMULE 53

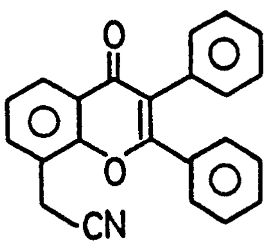

FORMULE 54

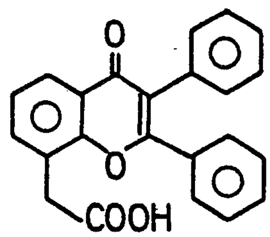

FORMULE 55

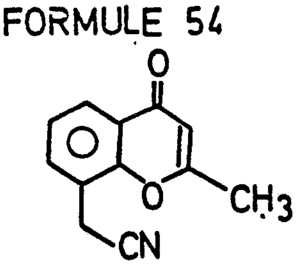

FORMULE 56

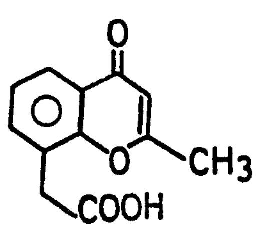

FORMULE 57